# EUROPÄISCHE PATENTSCHRIFT

(11) **EP 0 417 870 B1**
(45) Veröffentlichungstag und Bekanntmachung des Hinweises auf die Patenterteilung: **20.07.1994**
(21) Anmeldenummer: 90250214.5
(22) Anmeldetag: 20.08.1990
(51) Int. Cl.: C07C 331/28, A61K 49/02, A61K 43/00, C07C 215/50, C07C 215/86, C07C 251/38, C07C 323/32, C07D 333/36, C07D 233/91, C07D 309/12, C07D 405/14

(54) **Chelatbildner zur Komplexierung von radioaktiven Isotopen, deren Metallkomplexe sowie ihre Verwendung in Diagnostik und Therapie**
Chelates for complexation of radioactive isotopes, their metal complexes and their use in diagnosis and therapy
Chélates pour complexation d'isotopes radioactifs, leurs complexes et leur utilisation en diagnostic et thérapie

(30) Priorität: 11.09.1989 DE 3930674
(43) Veröffentlichungstag der Anmeldung: 20.03.1991
(73) Patentinhaber: INSTITUT FÜR DIAGNOSTIKFORSCHUNG GmbH AN DER FREIEN UNIVERSITÄT BERLIN, 14050 Berlin (DE)
(72) Erfinder: Neumeier, Reinhard, Dr., D-1000 Berlin 27 (DE); Kramp, Wolfgang, Dr., D-1000 Berlin 27 (DE); Mäcke, Helmut R., Dr., D-7850 Lörrach (DE)
(74) Vertreter: Wablat, Wolfgang, Dr.Dr.

(56) Entgegenhaltungen:
- EP-A- 0 123 504
- EP-A- 0 188 256
- FR-A- 2 331 549
- CHEMICAL ABSTRACTS, Band 105, 15. Dezember 1986, Seite 589, Zusammenfassung Nr.217818u, Columbus, Ohio, US; G.A. LAWRANCE et al.: "An unusual cleavage and macrocyclization in the zinc-aqueous reduction of[2-methyl]-2-nitro-N,N'-bis(pyridin-2-ylmethyl)propane-1,3-diamine]copper(II) cation. Crystal structure of the (6,13-dimethyl-1,4,8,11-tetraazacyclotetradecane-6,13-diamine)copper(II)perchlorate product"

## Beschreibung

Seit längerer Zeit werden radioaktive Metallionen, meist gebunden an einen Komplexbildner, für die in vivo-Diagnostik verwendet. Hiervon ist Technetium-99m (Tc-99m) aufgrund seiner für diese Zwecke nahezu idealen physikalischen Eigenschaften - gute Absorption der Strahlung in entsprechenden Detektionsgeräten (Gammakamera, SPECT-Geräte) gegenüber einer geringen Absorption im menschlichen Organismus und leichte Verfügbarkeit über einen Molybdän /Technetium-Generator - das am häufigsten in der klinischen Nuklearmedizin verwendete Radio -nuklid. Seine kurze Halbwertszeit von 6,02 h garantiert eine nur geringe Belastung des Patienten mit Gammastrahlung, zumal auch das Tochternuklid Technetium-99 nur eine vernachlässigbare Reststrahlung besitzt. Ein Nachteil des Technetiums ist allerdings seine komplizierte und noch nicht vollständig bekannte Komplex-Chemie. Technetium kann in einer Reihe von Oxidationsstufen (+7 bis - 1) vorliegen, die die pharmakologischen Eigenschaften durch Veränderung der Ladung eines Komplexes stark verändern können. Es ist deswegen nötig, Komplexe zu synthetisieren, die das Technetium in einer definierten Oxidationstufe binden und Redox-Reaktionen, die zu einer Redistribution des Pharmakons führen könnten, verhindern. Eine Reihe solcher Tc-99m-Komplexbildner sind bereits bekannt und werden klinisch angewendet. Bei neutralen Komplexen handelt es sich vielfach um Systeme, in denen das Tc-99m zwischen 2-4 Stickstoffatomen und 0-2 Schwefelatomen gebunden wird (N₂S₂,- N₃S- und Propylenaminoximkomplexe). Vielfach ist jedoch die ungenügende Stabilität dieser Tc-99m-Komplexe ein wesentlicher Nachteil (Hung, J.C. et al.; J. Nucl. Med. 29: 1568, 1988). In der klinischen Anwendung muß deswegen z.B. HMPAO (Hexamethyl-propylenaminoxim) kurz nach seiner Markierung mit Pertechnetat appliziert werden, damit der Anteil an Zerfallsprodukten, die die diagnostische Aussagekraft vermindern, nicht zu hoch wird. Eine Kopplung dieser Chelate bzw. Chelatbildner an andere, sich selektiv in Krankheitsherden anreichernde Substanzen ist nicht möglich. Daher verteilen sich die meisten der erwähnten Komplexe entsprechend der Durchblutung und/oder metabolischen Aktivität eines Organs (z.B. Europ. Patent Appl. 0 194 843), so daß z.B. nekrotische oder ischämische Regionen nach Infarkt oder Schlaganfall in einem Szintigramm dargestellt werden können.

Für eine erfolgreiche Diagnostik von Tumoren, neurologischen Erkrankungen oder Herz-Kreislauferkrankungen sind jedoch Substanzen vielversprechender, die molekulare Veränderungen der erkrankten Gewebe darstellen können, indem sie spezifisch an diese erkrankten Gewebe binden bzw. in deren Stoffwechsel eingeschleust werden. Die Erkenntnisse der biologischen und biochemischen Grundlagenforschung erlauben die Auswahl einer Reihe von Substanzen, die sich in Krankheitsherden selektiv anreichern: Diverse Tumoren entwickeln erhöhte oder erniedrigte Oberflächenkonzentrationen an Rezeptoren z.B. für Wachstumsfaktoren oder Steroidhormone (Sledge, G.W.; Adv. Cancer Res. 38:61-75 [1983]). Auch bei neurologischen Erkrankungen kommt es zu einer Veränderung der Konzentration von Rezeptoren für Neurotransmitter in bestimmten Bereichen des Hirns (Frost, J.J.; Trends Pharmacol. Sci. 7: 490-496 [1987]). Weiterhin zeigen erkrankte, geschädigte oder zu Tumorzellen transformierte Zellen oft starke Veränderungen ihres Metabolismus und einen Sauerstoffmangel im Innern des Tumors. Die Ausnutzung solcher physiologischer Besonderheiten kann der in vivo-Diagnostik dienen, indem z.B. Hormone, Wachstumsfaktoren, Neurotransmitter oder bestimmte Stoffwechselprodukte wie Fettsäuren, Saccharide, Peptide oder Aminosäuren an Chelatbildner für Tc-99m gekoppelt werden. Auch Substanzen wie Misonidazol (ein Radiosensitizer) bzw. andere in Abwesenheit von Sauerstoff sich zu Radikalen umsetzende Verbindungen können zur spezifischen Anreicherung von radioaktiven Isotopen und somit bildlichen Darstellung von Tumoren oder ischämischen Regionen herangezogen werden. Schließlich ist auch die Kopplung an monoklonale Antikörper möglich, die aufgrund ihrer hohen Spezifität zu einem vielversprechenden Instrument in der Tumordiagnostik geworden sind.

Für die Herstellung von Diagnostika nach dem beschriebenen Prinzip ist es notwendig, daß Chelatbildner für radioaktive Metallionen, insbesondere Tc-99m, an in erkrankten Geweben sich selektiv anreichernde Substanzen gekoppelt werden können.

Da die Isotope des Rheniums (Re-188 und Re-186) ähnliche chemische Eigenschaften wie Tc-99m besitzen, können die Chelatbildner auch zur Komplexierung dieser Isotope verwendet werden. Die genannten Re-Isotope sind ß-Strahler. Somit sind die sich selektiv anreichernden Substanzen mit Rhenium statt mit Technetium komplexiert auch in der Tumortherapie einsetzbar.

Die bisher bekannten Ansätze zur Kopplung von Chelatbildnern an sich selektiv anreichernde Substanzen können vielfach als nicht zufriedenstellend betrachtet werden. Werden die funktionellen Gruppen des Komplexbildners zur Bindung des Chelatbildners an ein solches Molekül benutzt, so kommt es häufig zu einer Abschwächung der Komplexstabilität, d.h. ein diagnostisch nicht tolerierbarer Anteil des Isotops wird aus dem Konjugat freigesetzt. (Brechbiel, M. W. et.al., Inorg. Chem. 25:2772 [1986]). Es ist deswegen notwendig bifunktionelle Komplexbildner herzustellen, d.h. Komplexbildner, die sowohl funktionelle Gruppen zur koordinativen Bindung des gewünschten Metallions als auch eine (andere) funktionelle Gruppe zur Bindung des sich selektiv anreichernden Moleküls tragen. Solche bifunktionellen Liganden ermöglichen eine spezifische, chemisch definierte Bindung von Technetium an verschiedenste biologische Materialien, auch dann, wenn ein sogenanntes Prelabeling durchgeführt wird. Da nach dieser Methode zuerst die Markierung mit Tc-99m und die Isolierung der Komplexe durchgeführt und erst in einem zweiten Schritt dieser Komplex mit einem sich selektiv anreicherndem Molekül verknüpft wird, erhält man die markierten Verbindungen mit einem hohen Reinheitsgrad.

Es wurden einige Chelatbildner gekoppelt an monoklonale Antikörper (z.B. Europ. Patent App. 0 247 866 und 0 188 256) oder Fettsäuren (Europ. Patent Appl. 0 200 492) beschrieben. Als Chelatbildner werden jedoch die bereits erwähnten N₂S₂-Systeme verwendet, die aufgrund ihrer geringen Stabilität wenig geeignet sind. Die etwas stabileren N₃S-Chelate zeigten gekoppelt an monoklonale Antikörper keinen so starken Verlust des Tc-99m aus den Konjugaten (J. Lister-James; J. Nucl. Med. 30:793 und Europ. Patent Appl. 0 284 071). In der EP-A-123 504 werden die bereits genannten Propylenaminoxim-Chelate beschrieben, die auch als Komplexbildner für Technetium geeignet sind. Die FR-A-2331549 beschreibt Verbindungen, die als Komplexbildner für Cu und Co geeignet sind.

Da sowohl die sich selektiv anreichernden Substanzen in ihren Eigenschaften, sowie auch die Mechanismen, nach denen sie angereichert werden, sehr unterschiedlich sind, ist es weiterhin notwendig, den kopplungsfähigen Chelatbildner variieren und den physiologischen Anforderungen des Kopplungspartners hinsichtlich Lipo- und Hydrophilie, Membranpermeabilität bzw. -impermeabilität etc. anpassen zu können.

Es besteht aus diesen Gründen ein dringlicher Bedarf an stabilen Komplexverbindungen, die gekoppelt oder fähig zur Kopplung an unterschiedliche sich selektiv anreichernde Verbindungen sind.

Aufgabe der Erfindung ist es somit, stabile Chelatbildner, die eine funktionelle Gruppe zur Kopplung an eine sich selektiv anreichernde Verbindung oder eine mit Hilfe dieser funktionellen Gruppe gekoppelte sich selektiv anreichernde Verbindung enthalten, zur Verfügung zu stellen.

Erfindungsgemäß wird diese Aufgabe durch die Verbindungen der allgemeinen Formel I gelöst, 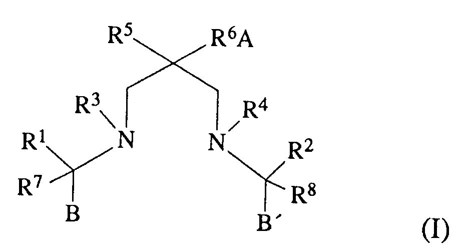
worin R¹, R² und R⁵ gleich oder unterschiedlich sind und für Wasserstoff oder einen gegebenenfalls mit einer Hydroxylgruppe substituierten C₁₋₆-Alkylrest,
R³ und R⁴ gleich oder unterschiedlich sind und für ein Wasserstoffatom, einen C₁₋₆-Alkyl- oder einen Amino(C₁₋₆)-alkylrest,
R⁶ für einen C₁₋₆-Alkylenrest,
R⁷ und R⁸ gleich oder unterschiedlich sind und für Wasserstoff oder einen C₁₋₆-Alkylrest und
B und B' gleich oder unterschiedlich sind und für einen mit 1-3 Hydroxylgruppen substituierten Phenyl- oder Naphthyl, 2-Mercaptophenyl-, Thienyl-, Pyrrolylrest stehen,
und A eine funktionelle Gruppe C darstellt,
wobei C für einen Amino-, einen Hydrazino- oder Hydrazido-, einen Carboxy-, einen C₂₋₆-Alkinyl- oder -Alkenyl-, einen Hydroxyl-, einen Aminophenyl-, einen Oxiranyl-, einen fluorierten Phenoxycarbonyl-, einen Nitril-, einen Isothiocyanatophenyl- oder einen gegebenenfalls mit einem Natriumsulfatrest substituierten Succinimidoxycarbonylrest steht,
oder eine - mit Hilfe der funktionellen Gruppe C gebundene - sich selektiv in Läsionen oder bestimmten Geweben anreichernde Verbindung T enthält,
wobei T für monoklonale Antikörper oder deren Fragmente, Hormone, Enzyme, Wachstumsfaktoren, Liganden für Zellmembranrezeptoren, Steroide, Neurotransmitter, Lipide, Sacharide, Aminosäuren und Oligopeptide, Biotin, sowie Radiosensitizer, wie z.B. Misonidazol steht,
deren Komplexe mit - zur Diagnostik und Tumortherapie geeigneten - radioaktiven Metallionen, sowie deren Salze mit anorganischen und organischen Säuren.

Erfindungsgemäß bevorzugt sind diejenigen Verbindungen gemäß Anspruch 1, in denen R¹, R², R⁷ und R⁸ Wasserstoffatome oder Methylreste bedeuten sowie diejenigen Verbindungen gemäß Anspruch 1, in denen die gegebenenfalls in A enthaltene funktionelle Gruppe C für einen Carboxyl-, einen Amino-, einen C₂₋₆-Alkenyl-, einen C₂₋₆-Alkinyl-, einen Nitril-, einen Oxiranyl-, einen Aminophenyl- oder einen Isothiocyanatophenylrest und die gegebenenfalls in A enthaltene sich selektiv anreichernde Verbindung T für monoklonale Antikörper, deren Fragmente, Biotin oder Misonidazol steht.

Überraschenderweise zeigten viele der synthetisierten und mit Tc-99m markierten Chelate eine höhere Stabilität als die vergleichbaren N₂S₂-, N₃S- und Propylenaminoxim-Chelate. Auch durch Kompetitionsversuche konnte festgestellt werden, daß die in dieser Erfindung beschriebenen Chelate Tc-99m besser als die vergleichbaren N₂S₂-, N₃S- und Propylenaminoxim-Chelaten komplexieren. Die in der Erfindung beschriebenen Chelate sind damit eindeutig besser für diagnostische und therapeutische Zwecke geeignet als die bisher bekannten Chelate.

Die Herstellung der Verbindungen gemäß Anspruch 1 erfolgt, indem man ein 1,3-Propandiamin der allgemeinen Formel II 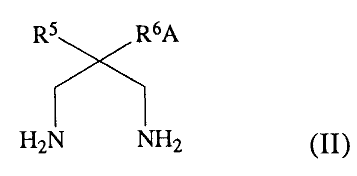
worin R⁵, R⁶ und A die oben genannten Bedeutungen haben, mit einer Verbindung der allgemeinen Formel III bzw. IV

B - R¹C=O (III)

B'- R²C=O (IV),

in denen R¹ = R², B = B' gilt und R¹, R², B und B' die oben genannten Bedeutungen haben, in einem polaren Lösungsmittel, vorzugsweise Ethanol, oder unter Verwendung eines Wasserabscheiders in einem unpolaren Lösungsmittel, vorzugsweise Benzol, bei Temperaturen von 25 -180 °C innerhalb von 6 Stunden bis 3 Tagen umsetzt,
die Iminofunktion in an sich bekannter Weise, vorzugsweise mit Natriumborhydrid in einem polaren Lösungsmittel, vorzugsweise einem Methanol/Wasser-Gemisch, bei Temperaturen von 25 - 100 °C innerhalb von 0,5 bis 24 Stunden, vorzugsweise 2 Stunden, reduziert,
oder
indem man ein Propandiamin der allgemeinen Formel II 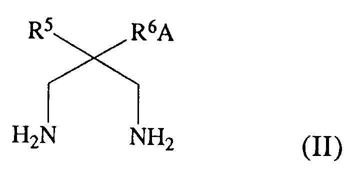
worin R⁵, R⁶ und A die oben genannten Bedeutungen haben, mit einer Verbindung der allgemeinen Formel V bzw. VI

B - CO-X (V)

B'- CO-X (VI),

in denen B = B' gilt und B und B' die oben genannten Bedeutungen haben, in B enthaltene Hydroxyl-, Mercapto- und Aminogruppen in geschützter Form vorliegen und X für ein Halogenatom, vorzugsweise für ein Chloratom steht,
oder substituierte Malonsäurehalogenide, vorzugsweise Malonsäurechloride der allgemeinen Formel VII, 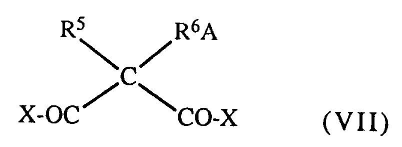
worin R⁵ und R⁶ die oben genannten Bedeutungen haben, X für ein Halogenatom steht und gegebenenfalls in B vorhandene Hydroxylgruppen in geschützter Form vorliegen, mit einem Amin der allgemeinen Formel VII bzw. IX 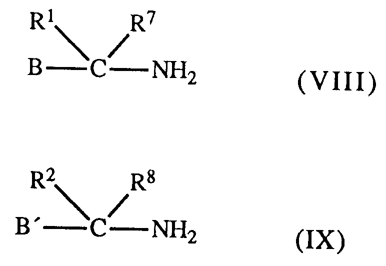
in denen R¹ = R², R⁷ = R⁸, B = B' gilt und R¹, R², R⁷, R⁸, B und B' die oben genannten Bedeutungen haben,
in einem aprotischen Lösungsmittel, vorzugsweise Dichlormethan, bei Temperaturen von 0 - 180 °C, vorzugsweise bei Raumtemperatur, innerhalb von 2 bis 24 Stunden, vorzugsweise 4 Stunden, unter Zusatz von Triethylamin umsetzt,
die Amidfunktion in an sich bekannter Weise, vorzugsweise mit Boran in THF oder mit Lithiumaluminiumhydrid in einem aprotischen Lösungsmittel, vorzugsweise Diethylether, bei Temperaturen von 25 - 150 °C innerhalb von 0,5 bis 24 Stunden, vorzugsweise 8 Stunden, zur entsprechenden Aminofunktion reduziert,
die Aminogruppen gegebenenfalls in an sich bekannter Weise alkyliert und vorhandene Schutzgruppen abspaltet
und die auf diesen gleichwertigen Wegen erhaltenen Verbindungen gegebenenfalls vor Generierung der freien funktionellen Gruppe C die vorhandenen Aminogruppen mit Schutzionen, z.B. als Cu-Komplex, schützt und anschließend die so erhaltenen Verbindungen gewünschtenfalls über die in A vorhandene funktionelle Gruppe C an sich selektiv anreichernde Verbindungen T koppelt und die Aromat-Substituenten B und B' gewünschtenfalls mit dem jeweils gewünschten radioaktiven Isotop komplexiert, wobei man vorher im Produkt gegebenenfalls vorhandene Schutzionen nach an sich literaturbekannten Methoden entfernt und die Reihenfolge der Schritte Komplexierung mit Technetium- oder Rhenium-Isotopen und Kopplung an T vertauscht werden kann.

Als Hydroxyschutzgruppen kommen z.B. die Benzyl-, 4-Methoxybenzyl-, 4-Nitrobenzyl-, Trityl-, Diphenylmethyl, Trimethylsilyl-, Dimethyl-t-butylsilyl- und Diphenyl-t-butylsilylgruppe infrage. Im Falle von Polyolen können die Hydroxygruppen auch in Form von Ketalen mit z.B. Aceton, Acetaldehyd, Cyclohexanon oder Benzaldehyd geschützt sein. Ferner können die Hydroxygruppen auch z.B. als THP-Ether, α-Alkoxyethylether, MEM-Ether oder als Ester mit aromatischen oder aliphatischen Carbonsäuren, wie z.B. Essigsäure oder Benzoesäure, vorliegen. Die Hydroxyschutzgruppen können nach den dem Fachmann bekannten Literaturmethoden, z.B. durch Hydrogenolyse, reduktive Spaltung mit Lithium / Ammoniak, Säurebehandlung der Ether und Ketale oder Alkalibehandlung der Ester freigesetzt werden (siehe z.B. "Protective Groups in Organic Synthesis", T.W. Greene, John Wiley and Sons 1981).

Als Aminoschutzgruppen kommen z.B. Trifluoracetyl-, t-Butoxycarbonyl-, 2,2,2-Trichloroethoxycarbonyl-, Benzoxycarbonyl- und Acetylgruppen infrage. Die Aminoschutzgruppen können nach literaturbekannten Methoden, z.B. durch basische oder saure Hydrolyse, reduktive Spaltung mit Zink in Essigsäure oder Hydrogenolyse abgespalten werden.

Als Mercaptoschutzgruppen kommen C₁₋₆-Alkylreste oder Benzylthioether infrage. Die Abspaltung der Mercaptoschutzgruppen erfolgt wahlweise mit Alkalialkylthiolaten, Alkalialkoholaten oder Alkalimetallen, vorzugsweise mit Natriummethylthiolat in einem polaren Lösungsmittel, vorzugsweise in HMPT, DMF oder Dimethylacetamid oder durch reduktive Spaltung mit Natrium / Ammoniak.

Nicht symmetrische Verbindungen (B ≠ B') lassen sich über kommerziell erhältliche, gemischte Malonsäureester darstellen, die eine selektive Umsetzung von zunächst nur einer der beiden unterschiedlichen Esterfunktionen zum Carbonsäureamid erlauben. Insbesondere sind dies Methyl-, Benzyl- oder t-Butylester, die sich nach literaturbekannten Methoden leicht neben anderen Carbonsäureestern, z.B. einem Ethylester, selektiv verseifen lassen. Die daraus resultierende freie Carbonsäurefunktion wird in an sich bekannter Weise in das entsprechende Carbonsäurechlorid überführt und anschließend mit einem gegebenenfalls substituierten primären Amin zu einem sekundären Carbonsäureamid umgesetzt. Die verbleibende Esterfunktion wird anschließend, wie bereits beschrieben, mit Ammoniak in ein primäres Carbonsäureamid überführt und anschließend die beiden unterschiedlichen Säureamidfunktionen in an sich bekannter Weise zu den unterschiedlich substituierten Aminen reduziert.

Die primäre Aminogruppe läßt sich, wie bereits beschrieben, mit einer Verbindung der Formel III oder IV zu dem entsprechenden Imin umsetzen. Die verbleibende sekundäre Aminogruppe kann anschließend unter reduktiven Alkylierungsbedingungen in an sich bekannter Weise mit einer Verbindung der Formel III oder IV umgesetzt werden, wobei gleichzeitig die Iminofunktion zum Amin reduziert wird.

Der Rest C ist dazu geeignet, eine stabile Verbindung zu Proteinen oder anderen sich selektiv anreichernden Molekülen herzustellen. Durch die entsprechende Wahl der funkionellen Gruppe C gelingt die Kopplung unter schonenden Reaktionsbedingungen, die die biologische Funktion und/oder Selektivität nicht beeinflussen.

Die Kopplung an die gewünschten Verbindungen erfolgt ebenfalls nach an sich bekannten Methoden, (z.B. Fritzberg et al.; J. Nucl. Med.: 26,7 [1987]), beispielsweise durch Reaktion der Gruppe C mit nucleophilen Gruppen des sich selektiv anreichernden Moleküls oder, wenn es sich bei der Gruppe C selbst um ein Nucleophil handelt, mit aktivierten Gruppen des sich selektiv anreichernden Moleküls.

Die Gruppe C repräsentiert jeden Substituenten, der einerseits eine funktionelle Gruppe darstellt, die eine Kopplung an ein sich selektiv anreicherndes Molekül unter milden Bedingungen erlaubt (z.B. durch Acylierung oder Amidierung) sowie jede aktivierte Gruppe, die mit nucleophilen Gruppen von Proteinen, Antikörpern, Hormonen oder anderen Biomolekülen reagieren kann, wie der Amino-, Phenol-, Sulfhydryl-, Formyl- oder Imidazol-Gruppe. Unter aktivierter Gruppe wird eine Funktion verstanden, die fähig ist, unter Bildung eines Konjugates mit einem nucleophilen Substituenten eines sich selektiv anreichernden Moleküls oder des Komplexliganden selbst in wässriger Lösung innerhalb einer angemessen kurzen Zeit, unter Reaktionsbedingungen, die weder Denaturierung noch Verlust der biologischen Aktivität zufolge haben, zu reagieren. Beispiele dafür sind Imidester, Alkylimidester, Amidoalkylimidester, Succinimidester, Acylsuccinimide, Phenolester, substituierte Phenolester, Tetrafluorphenolester, Anhydride, Hydrazide, Alkylhalogenide und Michael-Akzeptoren. C ist bevorzugt ein Monoanhydrid, Säurechlorid, Säurehydrazid, gemischtes Anhydrid, aktivierter Ester (wie Phenol- oder Imid-Ester), Nitren oder Isothiocyanat, insbesondere für die Kopplung mit nucleophilen Gruppen von Aminosäuren oder ein aliphatisches oder aromatisches primäres Amin für die Kopplung an Kohlenhydratresten von Proteinen.

Handelt es sich bei der Gruppe C selbst um ein Nucleophil, kann sie mit aktivierten Gruppen eines sich selektiv anreichernden Moleküls reagieren, wobei auch mit sogenannten "Cross-linking-reagents" umgesetzte Gruppen des Moleküls mit eingeschlossen sind, wie homobifunktionelle Imidoester, homobifunktionelle N-Hydroxysuccinimidester (NHS) und heterobifunktionelle "Cross-Linker", die unterschiedliche funktionelle Gruppen, wie NHS-Ester, Pyridyl-Disulfide und aktivierte Halogene, wie α-Keto-Halogenide enthalten. Solche Cross-Linker sind kommerziell erhältlich.

Als Kopplungspartner (= Verbindung T) sind u.a. verschiedene Biomoleküle vorgesehen: Liganden, die an spezifische Rezeptoren binden und so ein in ihrer Rezeptordichte verändertes Gewebe erkennen können; hierzu gehören u.a. Peptid- und Steroidhormone, Wachstumsfaktoren und Neurotransmitter. Mit Liganden für Steroidhormon-Rezeptoren wurde die Möglichkeit einer verbesserten Diagnostik von Brust- und Prostatacarcinomen aufgezeigt (S.J. Brandes & J.A. Katzenellenbogen, Nucl. Med. Biol. 15: 53, 1988). Verschiedentlich weisen Tumorzellen eine veränderte Dichte von Rezeptoren für Peptidhormone oder Wachstumsfaktoren, wie z.B. den "epidermal growth factor" (EGF) auf. Die Konzentrationsunterschiede konnten zur selektiven Anreicherung von Cytostatika in Tumorzellen genutzt werden (E. Aboud-Pirak et al., Proc. Natl. Acad. Sci. USA 86: 3778, 1989). Vielfach konnten mit Positronen-emittierenden Isotopen markierte Liganden für Neurorezeptoren zur Diagnostik verschiedener Hirnerkrankungen herangezogen werden (J.J. Forst, Trends in Pharmacol. Sci. 7: 490, 1987). Weitere Biomoleküle sind in den Metabolismus der Zellen einschleusbare Metaboliten, die einen veränderten Stoffwechsel erkennbar machen; hierzu gehören beispielsweise Fettsäuren, Saccharide, Peptide und Aminosäuren. Fettsäuren gekoppelt an die unstabileren N₂S₂-Chelatbildner wurden in der EPA 0 200 492 beschrieben. Andere Stoffwechselprodukte wie Saccharide (Dexoxyglucose), Lactat, Pyruvat und Aminosäuren (Leucin, Methylmethionin, Glycin) wurden mit Hilfe der PET-Technik zur bildlichen Darstellung von veränderten Stoffwechselvorgängen herangezogen (R. Weinreich, Swiss Med. 8, 10, 1986). Auch nicht-biologische Substanzen wie Misonidazol und seine Derivate, die sich in Geweben bzw. Gewebeteilen mit reduzierter Sauerstoffkonzentration irreversibel an Zellbestandteile binden, können zur spezifischen Anreicherung von radioaktiven Isotopen und somit bildlichen Darstellung von Tumoren oder ischämischen Regionen herangezogen werden (M.E. Shelton, J. Nucl. Med. 30: 351, 1989). Schließlich ist auch die Kopplung der bifunktionellen Chelatbildner an monoklonale Antikörper bzw. deren Fragmente möglich. Die Biotin enthaltenden erfindungsgemäßen Verbindungen ermöglichen die Bindung der radioaktiven Konjugate an Avidin- oder Streptavidin-haltige Substanzen. Dies kann genutzt werden, um Antikörper-Streptavidin-Konjugate am Tumor anzureichern und die radioaktive Biotin-haltige Komponente erst später zu applizieren, was zu einer verringerten Exposition des Patienten mit radioaktiver Strahlung führt (D.J. Hnatowich et al., J. Nucl. Med. 28: 1294, 1987). Durch Komplexierung der Konjugate mit Tc-99m bzw. Rhenium-Isotopen kann eine Diagnostik und Therapie von Tumoren ermöglicht werden.

Es ist unerheblich, ob eine Markierung der Chelatbildner mit Tc-99m oder einem Rhenium-Isotop vor oder nach der Kopplung an das sich selektiv anreichernde Molekül durchgeführt wird. Für eine Kopplung an das sich selektiv anreichernde Molekül nach einer Komplexierung ist jedoch Voraussetzung, daß die Umsetzung des radioaktiven Komplexes mit der sich anreichernden Verbindung schnell, unter schonenden Bedingungen und nahezu quantitativ abläuft, sowie keine anschließenden Aufreinigung erforderlich ist.

Die Herstellung der erfindungsgemäßen pharmazeutischen Mittel erfolgt in an sich bekannter Weise, in dem man die erfindungsgemäßen Komplexbildner unter Zusatz eines Reduktionsmittels, vorzugsweise Zinn-(II)-salzen wie -chlorid oder -tartrat - und gegebenenfalls unter Zugabe der in der Galenik üblichen Zusätze - in wäßrigem Medium löst und anschließend sterilfiltriert. Geeignete Zusätze sind beispielsweise physiologisch unbedenkliche Puffer (z.B. Tromethamin), geringe Zusätze von Elektrolyten (z.B. Natriumchlorid), Stabilisatoren (z.B. Gluconat, Phosphate oder Phosphonate). Das erfindungsgemäße pharmazeutische Mittel liegt in Form einer Lösung oder in lyophilisierter Form vor und wird kurz vor der Applikation mit einer Lösung Tc-99m-Pertechnetat, eluiert aus kommerziell erhältlichen Generatoren, oder einer Perrhenatlösung versetzt.

Bei der nuklearmedizinischen In-vivo-Anwendung werden die erfindungsgemäßen Mittel in Mengen von 1·10⁻⁵ bis 5·10⁴ nmol/kg Körpergewicht, vorzugsweise in Mengen zwischen 1·10⁻³ und 5·10² nmol/kg Körpergewicht dosiert. Ausgehend von einem mittleren Körpergewicht von 70 kg beträgt die Radioaktivitätsmenge für diagnostische Anwendungen zwischen 0,05 und 50 mCi, vorzugsweise 5 bis 30 mCi pro Applikation. Für therapeutische Anwendungen werden zwischen 5 und 500 mCi, vorzugsweise 10 - 350 mCi appliziert. Die Applikation wird normalerweise durch intravenöse, intraarterielle, peritoneale oder intratumorale Injektion von 0,1 bis 2 ml einer Lösung der erfindungsgemäßen Mittel erfolgen. Bevorzugt ist die intravenöse Applikation.

Die nachfolgenden Beispiele dienen der näheren Erläuterung des Erfindungsgegenstandes.

### Beispiel 1:

### 2-Methyl-2-(4-nitrobenzyl)-malonsäuredimethylester [1]

Es werden 11.5 g (0.5 mol) Natrium unter einem Stickstoffstrom in einen trockenen 1000 ml fassenden Dreihalskolben mit Rückflußkühler und Trockenrohr sowie Tropftrichter mit Druckausgleich gegeben. Anschließend werden vorsichtig 350 ml Methanol zugetropft und solange gerührt, bis das Natrium vollständig gelöst ist. In die noch warme Natriummethanolat-Lösung wird sehr langsam die methanolische Lösung von 87 g (0.5 mol) Methylmalonsäurediethylester zugetropft. Nach beendeter Zugabe wird noch 30 Minuten gerührt und dann portionsweise mit Hilfe eines Pulvertrichters das 4-Nitrobenzylbromid zugegeben. Nachdem sich alles gelöst hat wird zunächst 2 Stunden unter Rückfluß erhitzt und anschließend über Nacht bei Raumtemperatur gerührt. Das Lösungsmittel wird am Rotationsverdampfer abgezogen, der Rückstand mit Wasser versetzt und mehrmals mit Ethylacetat (je 250 mi) extrahiert. Die vereinigten organischen Extrakte werden mit gesättigter Kochsalzlösung gewaschen und über Natriumsulfat getrocknet. Nach Abziehen des Lösungsmittels verbleiben schwach gelbe Kristalle. Die Umkristallisation erfolgt aus Dimethylformamid (DMF)/ Wasser.
Schmelzpunkt: 94.5 - 95.0 °C
Ausbeute: 74%
¹H-NMR-Daten in DMSO/TMS
1.4 ppm (s,3H,Me); 3.5 ppm (s,2H,CH₂Ar); 3.8 ppm (s,6H,MeOOC); 7.2 ppm (d,2H, ArH); 8.2 ppm (d, 2H,ArH)

### 2-Methyl-2-(4-nitrobenzyl)-malonsäurediamid [2]

In einen 500 ml Rundkolben werden 5.0 g des Esters [1] eingewogen und in 200 ml Methanol gelöst. In diese methanolische Lösung wird bis zur Sättigung Ammoniak eingeleitet, anschließend die katalytische Menge Natrium zugegeben und nach Beendigung der Gasentwicklung der Kolben mit einem Absaugstück mit Luftballon verschlossen und mindestens 1 Woche bei Raumtemperatur stehen gelassen. Es fällt nach einigen Tagen ein weißer Niederschlag aus. Nachdem sämtliches Edukt umgesetzt ist (DC-Kontrolle), wird der ausgefallene Niederschlag abgesaugt und die verbleibende Lösung im Tiefkühlschrank auf -20°C abgekühlt und erneut abgesaugt. Die Mutterlauge wird am Rotationsverdampfer weiter eingeengt und der ausgefallende Niederschlag wieder abgesaugt. Die Umkristallisation der vereinigten Kristallfraktionen erfolgt aus Acetonitril. Das weiße kristalline Produkt wird im Vakuumexsikkator getrocknet.
Schmelzpunkt: 179 °C
Ausbeute: 91%
¹H-NMR-Daten in DMSO/TMS
1.2 ppm (s,3H,Me); 3.3 ppm (s,2H,CH₂Ar); 7.1 ppm (s(br),4H,CONH₂); 7.3 ppm (d,2H, ArH); 8.1 ppm (d,2H,ArH)

### 2-Methyl-2-(4-nitrobenzyl)-1,3-propandiamin [3]

Das Diamid [2] (5.0 g) wird unter Ausschluß von Luftfeuchtigkeit in 25 ml abs. Tetrahydrofuran in einem 250 ml Zweihalskolben mit Rückflußkühler und Trockenrohr sowie einem Septum suspendiert. Der Kolben wird in einem Eisbad heruntergekühlt und anschließend die Boran-Lösung in THF mit Hilfe einer 20 bzw. 50 ml-Einwegspritze durch das Septum zugegeben. Nachdem die erforderliche Menge der Boran-Lösung (80 ml einer 1 M Lösung in THF) zugegeben wurde, läßt man auf Raumtemperatur erwärmen. Nach 30 Minuten wird das Gemisch für 4 Stunden unter Rückfluß gekocht. Nach Abkühlung auf Raumtemperatur wird der Überschuß an Boran mit Wasser vorsichtig hydrolisiert (insgesamt 16 ml Wasser). Nach Beendigung der Gasentwicklung wird das Redaktionsgemisch quantitativ in einen 500 ml Rundkolben überführt und das Lösungsmittel am Rotationsverdampfer vorsichtig abgezogen, es verbleibt ein weißer Niederschlag. Dieser Niederschlag wird mit 125 ml 6 N HCl versetzt und 3 Stunden unter Rückfluß gekocht. Anschließend wird die Salzsäure am Rotationsverdampfer langsam abgezogen. Der Rückstand wird in der minimalen Menge Wasser aufgenommen (ca. 50-70 ml) und auf eine präparierte Ionenaustauschersäule (stark basisch) gegeben und das reine Amin mit destilliertem Wasser eluiert. Das freie Diamin wird aufgefangen (pH-Kontrolle) und die vereinigten Fraktionen werden am Rotationsverdampfer eingeengt. Als Rückstand verbleibt ein schwach gelbes Öl.
Schmelzpunkt (Hydrochlorid): 270 °C
Ausbeute: 80%
¹H-NMR-Daten in DMSO/TMS
1.0 ppm (s,3H,Me); 2.9 ppm (m,4H,CH₂NH₂); 3.0 ppm (s,2H,CH₂Ar); 7.5 ppm (d,2H,ArH); 8.2 ppm (d,2H,ArH); 8.5 ppm (s(br),4H,NH₂)

### 2-Methyl-2-(4'-nitrobenzyl)-N,N'-propylen-bis(salicyliden-imin) [4]

Zu einer Lösung von 5.6 g Diamin [3] in 75 ml Ethanol abs. wird unter Rühren eine Lösung von 5.5 g Salicylaldehyd in 75 ml Ethanol abs. zugetropft. Die resultierende Lösung verfärbt sich langsam intensiv gelb. Es wird noch 3 Stunden bei Raumtemperatur gerührt, dabei fällt ein gelber Niederschlag aus, der nach Abkühlen auf -20 °C abfiltriert wird. Insgesamt werden 7.5 g des Diimins erhalten.
Schmelzpunkt: 103 °C
Ausbeute: 77%
¹H-NMR-Daten in CDCl₃/TMS
1,1 ppm (s,3H,Me); 3,0 ppm (s,2H,CH₂Ar); 3,5 ppm (m,4H,CH₂N=C); 6,9 ppm (m,4H,ArH); 7,3-7,4 ppm (m,8H,ArH); 8,2 ppm (d,2H,ArH); 8,4 ppm (s,2H,CH=N)

### 2-Methyl-2-(4'-nitrobenzyl)-N,N'-propylen-bis(salicylidenamin) [5]

Zu einer Lösung von 2 g Diimin [4] in 50 ml Ethanol werden unter Rühren 300 mg Natriumborhydrid zugegeben und 2 Stunden bei 0 °C gerührt. Anschließend werden 20 ml Wasser zugetropft und 1 Stunde bei Raumtemperatur gerührt, danach das Lösungsmittel am Rotationsverdampfer abgezogen und der Rückstand in Wasser aufgenommen. Mit einer gesättigten Kaliumcarbonatlösung wird auf pH 11 gebracht und der Niederschlag in Chloroform aufgenommen. Nach Trocknen über Natriumsulfat und abziehen des Lösungsmittels verbleibt ein kristalliner Rückstand. Nach Umkristallisation aus Acetonitril verbleiben weiße Kristalle.
Schmelzpunkt: 151 °C
Ausbeute: 81%
¹H-NMR-Daten in CDCl₃/TMS
0,9 ppm (s,3H,Me); 2,5 ppm (m,4H,CH₂NH); 2,8 ppm (s,2H,CH₂Ar); 3,9ppm (m,4H,ArCH₂NH); 6,8 ppm (m,4H,ArH); 7,0-7,3 ppm (m,6H,ArH); 8,2 ppm (d,2H,ArH)

### 2-Methyl-2-(4'-aminobenzyl)-N,N'-propylen-bis(salicyliden-amin) [6]

In einem 50 ml Zweihalskolben werden 20 mg 10% Pd/C in 25 ml MeOH suspendiert und mit Wasserstoff gesättigt. Anschließend wird die Lösung von 218 mg [5] (0.5 mmol) in 4 ml Methanol und 833 »l 6N Salzsäure mit einer 5 ml Einwegspritze durch ein Septum zugegeben. Nach Beendigung der Wasserstoffaufnahme wird vom Katalysator abgetrennt und das Lösungsmittel im Vakuum abgezogen. Es verbleiben weiße Kristalle. Die Umkristallisation erfolgt aus Methanol.
Ausbeute: 73%
¹H-NMR-Daten in DMSO/TMS
1,0 ppm (s,3H,Me); 2,5 ppm (m,4H,CH₂NH); 2,8 ppm (s,2H,CH₂Ar); 4,1ppm (m,4H,ArCH₂NH); 6,8-7,5 ppm (m,12H,ArH)

### 2-Methyl-2-(4-isothiocyanatobenzyl)-N,N'-propylen-bis-(salicylidenamin) [7]

Eine 85%ige Lösung von Thiophosgen in Tetrachlorkohlenstoff (0,2 ml, 2,23 mmol) wird zu der Lösung von Verbindung [6] (0,16 mmol) in 4 ml Salzsäure (3 M) gegeben. Das Reaktionsgemisch wird 6 Stunden bei Raumtemperatur gerührt und dann im Vakuum bis zur Trockene eingeengt.
Ausbeute: 87%
¹H-NMR-Daten in DMSO/TMS
1,0 ppm (s,3H,Me); 2,8 ppm (m,4H,CH₂NH); 3,0 ppm (s,2H,CH₂Ar); 4,1ppm (m,4H,ArCH₂NH); 6,8-7,4 ppm (m,12H,ArH)

### Beispiel 2:

### 2-(4-Nitrobenzyl)-malonsäurediethylester [8]

Es werden 21.4 g Lithiumdiisopropylamid zu 210 ml wasserfreiem Tetrahydrofuran unter einem Stickstoffstrom in einem trockenen Dreihalskolben mit Trockenrohr und Tropftrichter mit Druckausgleich gegeben. Anschließend werden bei Raumtemperatur 58.0 g Malonsäurediethylester in 100 ml wasserfreiem Tetrahydrofuran innerhalb von 40 Minuten zugetropft. Nach 30 Minuten wird die Reaktionslösung auf -62 °C abgekühlt und 39.1 g 4-Nitrobenzylbromid in Tetrahydrofuran langsam unter starkem Rühren zugetropft, eine weitere Stunde bei -62 °C gerührt und anschließend wird der gebildete Niederschlag in der Kälte abfiltriert. Das Filtrat wird am Rotationsverdampfer eingeengt, der Rückstand in 300 ml Ethanol bei 65 °C in Lösung gebracht und vom unlöslichen Rückstand abgetrennt. Nach dem Abkühlen erhält man 34.7 g schwach gelbliche Kristalle.
Schmelzpunkt: 57 - 58 °C
Ausbeute: 65%
¹H-NMR-Daten in CDCl₃/TMS
1.2 ppm (t,6H,CH₂CH₃); 3.3 ppm (d,2H,CH₂Ar); 3.6 ppm (t,1H,CH); 4.1 ppm (q,4H,CH₂CH₃); 7.4 ppm (d,2H,ArH); 8.1 ppm (d,2H, ArH)

### 2-(4-Nitrobenzyl)-malonsäurediamid [9]

Diese Verbindung wird entsprechend der Verbindung [2] dargestellt.
Schmelzpunkt: 225 - 228 °C
Ausbeute 98%
¹H-NMR-Daten in DMSO/TMS
3.1 ppm (d,2H,CH₂Ar); 3.4 ppm (t,1H,CH); 7.1 ppm (s,4H,NH₂); 7.4 ppm (d,2H,ArH); 8.2 ppm (d,2H,ArH)

### 2-(4-Nitrobenzyl)-1,3-propandiamin [10]

Diese Verbindung wird entsprechend der Verbindung [3] dargestellt.
Schmelzpunkt (Hydrochlorid): 273-275 °C
¹H-NMR-Daten in D₂O
1.3 ppm (t,1H,CH); 3.1 ppm (d,4H,CH₂NH₂); 3.2 ppm (d,2H,CH₂Ar); 7.5 ppm (d,2H,ArH); 8.2 ppm (d,2H,ArH)

### 2-(4'-Nitrobenzyl)-N,N'-propylen-bis (salicylidenimin) [11]

Diese Verbindung wird entsprechend der Verbindung [4] dargestellt.
Schmelzpunkt: 75 °C
Ausbeute: 68%
¹H-NMR-Daten in CDCl₃/TMS
1.5 ppm (m,1H,CH); 3,2 ppm (d,2H,CH₂Ar); 3,5 ppm (m,4H,CH₂N=C); 7.1 ppm (m,4H,ArH); 7,3-7,4 ppm (m,8H,ArH); 8,2 ppm (d,2H,ArH); 8,4 ppm (s,2H,CH=N)

### 2-(4'-Nitrobenzyl)-N,N'-propylen-bis-(salicylidenamin) [12]

Diese Verbindung wird entsprechend der Verbindung [5] dargestellt.
Ausbeute: 90%
¹H-NMR-Daten in CDCl₃/TMS
1.6 ppm (m,1H,CH); 2,5 ppm (m,4H,CH₂NH); 2,8 ppm (d,2H,CH₂Ar); 3,9 ppm (m,4H,ArCH₂NH); 6,9 ppm (m,4H,ArH); 7,1-7,3 ppm (m,6H, ArH); 8,1 ppm (d,2H,ArH)

### 2-(4'-Aminobenzyl)-N,N'-propylen-bis(salicylidenamin) [13]

Diese Verbindung wird entsprechend der Verbindung [6] dargestellt.
Ausbeute: 84 %
¹H-NMR-Daten in DMSO/TMS
1.5 ppm (m,1H,CH); 2,5 ppm (m,4H,CH₂NH₂); 2,7 ppm (t,2H,CH₂Ar); 4,1 ppm (m,4H,ArCH₂NH); 6,8-7,5 ppm (m,12H,ArH)

### Beispiel 3

### 1,7-Bis(2,3-Dihydroxyphenyl)-4-(4'-nitrobenzyl)-2,6-diazahepta-1,6-dien [14]

Diese Verbindung wird entsprechend der Verbindung [4] dargestellt.
Ausbeute: 76%
¹H-NMR-Daten in CDCl₃/TMS
1.5 ppm (m,1H,CH); 3,1 ppm (d,2H,CH₂Ar); 3,5 ppm (m,4H,CH₂N=C); 6,8-7,0ppm (m,8H,ArH); 7.4 ppm (m,2H,ArH); 8,2 ppm (d,2H,ArH); 8,4ppm (s,2H,CH=N)

### 1,7-Bis(2,3-Dihydroxyphenyl)-4-(4'-nitrobenzyl)-2,6-diazaheptan [15]

Diese Verbindung wird entsprechend der Verbindung [5] dargestellt.
Ausbeute: 38%
¹H-NMR-Daten in CDCl₃/TMS
1.6 ppm (m,1H,CH); 2,5 ppm (m,4H,CH₂NH); 2,9 ppm (s,2H,CH₂Ar); 4,0 ppm (m,4H,ArCH₂NH); 6,5-7,3 ppm (m,8H,ArH); 8,2 ppm (d,2H,ArH)

### 1,7-Bis(2,3-Dihydroxyphenyl)-4-(4-aminobenzyl)-2,6-diazaheptan [16]

Es werden 250 mg der Nitroverbindung [15] in 30ml 50%igem Methanol (pH 11) gelöst und bei Raumtemperatur unter Zusatz von 25 mg Pd/Alox hydriert. Nach Abtrennen des Katalysators und Einengen verbleiben 60 mg eines kristallinen Festkörpers.
Ausbeute: 27%
¹H-NMR-Daten in DMSO/TMS
1.5 ppm (m,1H,CH); 2,5 ppm (m,4H,CH₂NH₂); 2,7 ppm (t,2H,CH₂Ar); 4,1 ppm (m,4H,ArCH₂NH); 6,6-7,5 ppm (m,12H,ArH)

### Beispiel 4

### 2-Methyl-2-[4'-(tetrahydro-2-pyranyloxy)butyl]-malonsäuredimethylester [22]

7,9 g (0.3 mol) Natrium werden in einem 500 ml Dreihalskolben mit Rückflußkühler und Trockenrohr sowie Tropftrichter mit Druckausgleich unter einem Stickstoffstrom gegeben. Anschließend werden vorsichtig 250 ml Methanol wasserfrei zugetropft und solange gerührt, bis das Natrium vollständig gelöst ist. In die noch warme Natriummethanolat-Lösung wird sehr langsam die methanolische Lösung von 101 g (0.58 mol) 2-Methylmalonsäurediethylesters zugetropft. Nach beendeter Zugabe wird noch 1 Stunde unter Rückfluß gekocht und dann langsam 0.5 mol 1-Chlor-4-tetrahydropyranyloxybutan zugetropft. Nach beendeter Zugabe wird über Nacht unter Rückfluß gekocht. Das Lösungsmittel wird am Rotationsverdampfer abgezogen, der Rückstand mit Wasser versetzt und mehrmals mit Ethylacetat extrahiert. Die vereinigten organischen Extrakte werden mit gesättigter Kochsalzlösung gewaschen und über Natriumsulfat getrocknet. Nach Abziehen des Lösungsmittels verbleibt ein farbloses Öl. Nach Destillation im Vakuum verbleiben 70,7 g des substituierten Malonesters.
Ausbeute: 78%
¹H-NMR-Daten in CDCl₃/TMS
1,4 ppm (s,3H,Me); 1,2-2,0 ppm (m,12H,CH₂); 3,5-3,8 ppm (m,4H, OTHP); 3,7 ppm (s,6H,COOMe); 4,6 ppm (t, 1H,OTHP)

### 2-Methyl-2-[4'-(tetrahydro-2-pyranyloxy)butyl]-malonsäurediamid [23]

In einem 1000 ml Rundkolben werden 40 g des substituierten Malonesters [22] eingewogen und mit 700 ml Methanol versetzt. Diese Lösung wird auf -40 °C abgekühlt und bis zur Sättigung mit Ammoniakgas versetzt, anschließend mit einer katalytischen Menge Natrium versetzt und bei dieser Temperatur 4 Stunden gerührt. Nach Erwärmen auf Raumtemperatur wird der ausgefallende Niederschlag abgesaugt und die verbleibende Lösung im Tiefkühlschrank auf -20 °C abgekühlt und erneut abgesaugt. Die Um-kristallisation der vereinigten Kristallfraktionen erfolgt aus Acetonitril.
Ausbeute: 86%
¹H-NMR-Daten in DMSO/TMS
1,3 ppm (s,3H,Me); 1.2-2.0 ppm (m,12H,CH₂); 3.1-4,0 ppm (m,4H, OTHP); 4.5 ppm (t,1H,OTHP); 7.1 ppm (s(br),4H,CONH₂)

### 2-Methyl-2-[4'-(tetrahydro-2-pyranyloxy)butyl]-1,3-propandiamin [24]

In einem 1000 ml fassenden Rundkolben mit Rückflußkühler und Tropftrichter werden unter Feuchtigkeitsausschluß zu 400 ml THF abs. 6.8 g (0.18 mol) Lithium-aluminiumhydrid gegeben. Anschließend werden 16.3 g (0.06 mol) des substituierten Malonsäureamids [23] in 100 ml THF abs. langsam zugetropft und 4 Stunden unter Rückfluß erhitzt, dann auf 0 °C abgekühlt, vorsichtig 8 ml Wasser zugegeben, mit 6 ml einer 20%igen Natronlauge und erneut mit 30 ml Wasser versetzt. Es wird abfiltriert und der verbleibende Niederschlag zweimal mit THF ausgekocht. Die vereinigten Filtrate werden mit gesättigter Kochsalzlösung gewaschen, über Natriumsulfat getrocknet, das Lösungsmittel am Rotationsverdampfer abgezogen und der Rückstand im Vakuum destilliert.
Ausbeute: 64%
¹H-NMR-Daten in CDCl₃/TMS
0,9 ppm (s,3H,Me); 1,2-1,9 ppm (m,12H,CH₂); 2,7 ppm (m,4H, CH₂NH₂); 3,3-3,8 ppm (m,4H,OTHP); 4,5 ppm (t,1H,OTHP)

### 2-Methyl-2-(4-tetrahydropyranyloxybutyl)-N,N'-propylen-bis(salicylidenimin) [25]

Diese Verbindung wird entsprechend der Verbindung [4] dargestellt.
Ausbeute: 77 %
¹H-NMR-Daten in CDCl₃/TMS
1,0 ppm (s,3H,Me); 1,2-1,9 ppm (m,12H,OTHP+CH₂); 3,2-3,7 ppm (m, 8H,OTHP+CH₂N=C); 4,5 ppm (t,1H,OTHP); 7,0 ppm (m,4H,ArH); 7,3-7,5 ppm (m,4H,ArH); 8,3 ppm (s,2H,CH=N)

### 2-Methyl-2-(4-tetrahydropyranyloxybutyl)-N,N'-propylen-bis(salicylidenamin) [26]

Diese Verbindung wird entsprechend der Verbindung [5] dargestellt.
Ausbeute: 89 %
¹H-NMR-Daten in DMSO/TMS
1,0 ppm (s,3H,Me); 1,2-1,8 ppm (m,12H,OTHP+CH₂); 2,8-3,1 ppm (m, 4H,CH₂NH₂); 3,3-3,9 ppm (m,8H,OTHP+ArCH₂NH); 4,5 ppm (t,1H, OTHP); 7,0 ppm (m,4H,ArH); 7,3-7,5 ppm (m,4H,ArH)

### Cu-2-Methyl-2-(4-tetrahydropyranyloxybutyl)-N,N'-propylen-bis(salicylidenamin) [27]

Zu einer Suspension von 913 mg [26] (2 mmol) in 50 ml Methanol werden 400 mg Kupferacetat (2 mmol) in 20 ml Methanol getropft und bei Raumtemperatur gerührt. Nach Abziehen des Lösungsmittels am Rotationsverdampfer und Trocknung an der Vakuumpumpe verbleibt ein öliger Rückstand. Nach Umkristallisation aus Pyridin/Chloroform verbleibt ein kristalliner Rückstand.
Ausbeute: 76%

### Cu-2-Methyl-2-(4-hydroxybutyl)-N,N'-propylen-bis(salicylidenamin) [28]

In eine Lösung von 0,3 ml konzentrierter Schwefelsäure in 5ml Methanol wird die Lösung von 877 mg [27] (1,7 mmol) in 5 ml Methanol getropft und 15 Stunden bei Raumtemperatur gerührt. Nach Abkühlung auf 0°C wird vorsichtig unter Rühren und Kühlen mit einer Natriumcarbonat neutralisiert. Nach dem Abziehen des Lösungsmittels wird der Rückstand mit Ether extrahiert, mit gesättigter Kochsalzlösung gewaschen und über Natriumsulfat getrocknet.
Ausbeute: 68%

### Beispiel 5

### 2-Allyl-malonsäurediamid [35]

Diese Verbindung wird entsprechend der Verbindung [2] dargestellt.
Schmelzpunkt: 168.5 - 169 °C
Ausbeute: 94%
¹H-NMR-Daten in DMSO/TMS
2,4 ppm (m,2H,CH₂CH=CH₂); 3.0 ppm (t,1H,CH); 5.0 ppm (m,2H, CH=CH₂); 5.7 ppm (m,1H,CH₂CH=CH₂); 7.0 ppm (s br,2H, CONH₂); 7.3 ppm (s br,2H,CONH₂)

### 2-Allyl-1,3-propandiamin [36]

Zu einer Suspension von 1.52 g Lithiumaluminiumhydrid in 50 ml wasserfreiem Tetrahydrofuran wird unter Eiskühlung langsam die Suspension von 3.12 g des Diamids [35] in 50 ml wasserfreiem Tetrahydrofuran zugegeben. Man läßt langsam auf Raumtemperatur erwärmen und erhitzt das Reaktionsgemisch anschließend 8 Stunden unter Rückfluß. Nach Abkühlung auf Raumtemperatur werden tropfenweise 10 ml Wasser, dann 10 ml 10%ige Kaliumhydroxidlösung und wiederum 10 ml Wasser zugegeben. Ausgefallenes Aluminiumhydroxid wird abfiltriert und zweimal mit je 50 ml Tetrahydrofuran kurz ausgekocht. Die vereinigten Filtrate werden mit gesättigter Kochsalzlösung gewaschen, über Natriumsulfat getrocknet und der Rückstand nach Abdampfen des Lösungsmittels im Vakuum destilliert.
Ausbeute: 65%
¹H-NMR-Daten in CDCl₃/TMS
1.9 ppm (m,1H,CH); 2.1 ppm (d,2H,CH₂CH=CH₂); 2,7 ppm (d,4H, CH₂NH); 5.1 ppm (m,2H,CH₂CH=CH₂); 5.8 ppm (m,1H,CH₂CH=CH₂)

### 2-Allyl-N,N'-propylen-bis(salicylidenimin) [37]

Diese Verbindung wird entsprechend der Verbindung [4] dargestellt.
Ausbeute: 83 %
¹H-NMR-Daten in CDCl₃/TMS
1,5 ppm(m,1H,CH); 2,2-2,4 ppm (m,2H,CH₂CH=CH₂); 3,5 ppm (m,4H,CH₂N=C); 4,9 ppm (m,2H,CH=CH₂); 5,6 ppm (m, 1H, CH=CH₂); 6,8-7,1 ppm (m,8H,ArH); 8,4 ppm (s,2H,CH=N)

### 2-(3-Brom-2-hydroxypropyl)-N,N'-propylen-bis(salicylidenimin) [38]

Eine Lösung von 3.22g [37] (10 mmol) in 50 ml THF/Wasser 6:1 wird mit 1.78g NBS (10 mmol) versetzt und bei Raumtemperatur unter Lichtausschluß 2 Tage gerührt. Das Gemisch wird in 250 ml Wasser gegossen und mehrmals mit je 50 ml Essigester extrahiert, getrocknet, eingeengt und der Rückstand aus Acetonitril umkristallisiert.
Ausbeute: 32%
¹H-NMR-Daten in CDCl₃/TMS
1.5 ppm (m,3H,CH+CH₂CHOH); 3.5 ppm (m,4H,CH₂N=C); 3,8 ppm (d,2H,CH₂Br); 4,1 ppm (m,1H,CHOH); 6,9 ppm (m,4H,ArH); 7,3 ppm (m,4H,ArH); 8,4 ppm (s,2H, CH=N)

### 2-(2,3-Epoxypropyl)-N,N'-propylen-bis(salicylidenimin) [39]

Eine Lösung von 7,6 g [38] (18 mmol) in 100 ml Methanol wird zu einer Lösung von 5,4 g Kaliumcarbonat in 15 ml Wasser gegeben und 1 Stunde bei Raumtemperatur gerührt. Es wird filtriert, eingeengt, der Rückstand mit 30 ml Wasser verdünnt, mit Chloroform extrahiert, über Natriumsulfat getrocknet und eingeengt.
Ausbeute: 76%
¹H-NMR-Daten in CDCl₃/TMS
1,5 ppm (m,3H,CH+CH₂CHOR); 2,6 ppm (m,2H,Epoxid); 3,1 ppm (m, 1H,Epoxid); 3.5 ppm (m,4H,CH₂N=C); 6,9 ppm (m,4H,ArH); 7,3 ppm (m,4H,ArH); 8,4 ppm (s, 2H,CH=N)

### 2-[2-Hydroxy-3-(2-nitroimidazolyl)-propyl]-N,N'-propylen-bis(salicylidenimin) [40]

In einem 10 ml Rundkolben wird das Gemisch aus 250 mg 2-Nitroimidazol (2,2 mmol), 475 mg 1,8-Bis-(dimethylamino)-naphthalin (2,2 mmol) und 920 mg [39] (2,2 mmol) in 5 ml DMSO unter Rühren 24 Stunden auf 80 °C erwärmt. Nach dem Abkühlen wird mit 50 ml Wasser verdünnt, mehrmals mit Essigester extrahiert, anschließend mit verdünnter Salzsäure und Wasser gewaschen, über Natriumsulfat getrocknet und eingeengt. Der Rückstand wird aus Methanol/Wasser umkristallisiert.
Ausbeute: 28%
¹H-NMR-Daten in CDCl₃/TMS
1,4-1,5 ppm (m,3H,CH+CH₂CHOH); 2,9 ppm (m,2H,CHOH-CH₂-NR₂); 3,5 ppm (m,4H,CH₂N=C); 4,0 ppm (m,1H,CHOH); 6,9-7,4 ppm (m,10H,ArH); 8.4 ppm (s,2H,CH=N)

### 2-[2-Hydroxy-3-(2-nitroimidazolyl)-propyl]-N,N'-propylen-bis(salicylidenamin) [41]

Diese Verbindung wird entsprechend der Verbindung [4] dargestellt.
Ausbeute: 63 %
¹H-NMR-Daten in CDCl₃/TMS
1,4-1,5 ppm (m,3H,CH+CH₂CHOH); 2,5 ppm (m,4H,CH₂NH); 2,8 ppm (m,2H,CHOH-CH₂-NR₂); 3,9-4,1 ppm (m,5H,CHOH+ArCH₂NH); 6,9-7,4 ppm (m,10H, ArH)

### Beispiel 6

### 2-Allyl-2-methyl-malonsäurediethylester [42]

Diese Verbindung wird entsprechend der Verbindung [1] dargestellt.
Ausbeute: 76%
Siedepunkt: 25_{mbar} 106-107 °C
¹H-NMR-Daten in CDCl₃/TMS
1.3 ppm (t,6H,COOCH₂CH₃); 1.4 ppm (s,3H,Me); 2.6 ppm (d,2H, CH₂CH=CH₂); 4,2 ppm (q,4H,COOCH₂CH₃); 5.0 ppm (d br,1H, CH₂CH=CH₂); 5.1 ppm (d br,1H,CH₂CH=CH₂); 5.7 ppm (m,1H, CH₂CH=CH₂)

### 2-Allyl-2-methyl-malonsäurediamid [43]

Diese Verbindung wird entsprechend der Verbindung [2] dargestellt.
Ausbeute: 89%
¹H-NMR-Daten in DMSO/TMS
1.3 ppm (s,3H,Me); 2.2 ppm (d,2H,CH₂CH=CH₂); 4.9 ppm (d br, 1H, CH₂CH=CH₂); 5.0 ppm (d br,1H,CH₂CH=CH₂); 5.8 ppm (m,1H, CH₂CH=CH₂)

### 2-Allyl-2-methyl-1,3-propandiamin [44]

Diese Verbindung wird entsprechend der Verbindung [36] dargestellt.
Ausbeute: 62 %
¹H-NMR-Daten in CDCl₃/TMS
1,0 ppm (s,3H,Me); 2,2 ppm (d,2H,CH₂CH=CH₂); 2,5 ppm (m,4H, CH₂NH); 4.9 ppm (d br,1H,CH₂CH=CH₂); 5.0 ppm (d br,1H, CH₂CH=CH₂); 5.8 ppm (m,1H, CH₂CH=CH₂)

### 2-Allyl-2-methyl-N,N'-propylen-bis(salicylidenimin) [45]

Diese Verbindung wird entsprechend der Verbindung [4] dargestellt.
Ausbeute: 76%
¹H-NMR-Daten in CDCl₃/TMS
1,0 ppm (s,3H,Me); 2,2 ppm (d,2H,CH₂CH=CH₂); 3,7 ppm (m,4H, CH₂N); 5.1 ppm (m,2H,CH₂CH=CH₂); 5.8 ppm (m,1H,CH₂CH=CH₂); 6,9-7,4 ppm (8H, ArH); 8,4 ppm (s, 2H, CH=N); 13,5 ppm (s, 2H, ArOH)

### 2-Allyl-2-methyl-N,N'-propylen-bis(salicylidenamin) [46]

Diese Verbindung wird entsprechend der Verbindung [5] dargestellt.
Ausbeute: 73%
¹H-NMR-Daten in CDCl₃/TMS
1,1 ppm (s,3H,Me); 2,3 ppm (d,2H,CH₂CH=CH₂); 2,8 ppm (m,4H, CH₂NH); 4,1 ppm (m, 4H, ArCH₂NH); 5.1 ppm (m,2H,CH₂CH=CH₂); 5.8 ppm (m,1H,CH₂CH=CH₂); 6,8-7,4 ppm (8H, ArH); 13,5 ppm (s, 2H, ArOH)

### Beispiel 7

### 1,7-Bis(2-Hydroxynaphthyl)-4-methyl-4-(4-nitrobenzyl)-2,6,diazahepta-1,6-dien [47]

Diese Verbindung wird entsprechend der Verbindung [4] dargestellt
Ausbeute: 80%
¹H-NMR-Daten in CDCl₃/TMS
1,2 ppm (s,3H,Me); 3,0 ppm (s,2H,CH₂Ar); 3,8 ppm (m,4H,CH₂N=C); 7,0 ppm (d,2H,ArH); 7,3-7,4 ppm (m,6H,ArH); 7,6-7,9 ppm (m,6H,ArH); 8,3 ppm (d,2H, ArH); 8,9 ppm (s,2H,CH=N)

### 1,7-Bis(2-Hydroxynaphthyl)-4-methyl-4-(4-nitrobenzyl)-2,6-diazaheptan [48]

Diese Verbindung wird entsprechend der Verbindung [5] dargestellt
Ausbeute: 52 %
¹H-NMR-Daten in CDCl₃/TMS
1,0 ppm (s,3H,Me); 2,6 ppm (m,4H,CH₂NH); 2,9 ppm (s,2H,CH₂Ar); 4,0 ppm (m,4H,ArCH₂NH); 6,9-7,4 ppm (m,8H,ArH); 7,6-7,9 ppm (m,6H,ArH); 8,3 ppm (d,2H,ArH)

### 1,7-Bis(2-Hydroxynaphthyl)-4-methyl-4-(4-aminobenzyl)-2,6-diazaheptan [49]

Diese Verbindung wird entsprechend der Verbindung [6] dargestellt
Ausbeute: 72 %
¹H-NMR-Daten in CDCl₃/TMS
1,1 ppm (s,3H,Me); 2,6 ppm (m,4H,CH₂NH); 2,9 ppm (s,2H,CH₂Ar); 3,9ppm (m,4H,ArCH₂NH); 6,9-7,4 ppm (m,8H,ArH); 7,6-7,9 ppm (m, 6H,ArH); 8,3 ppm (d,2H,ArH)

### Beispiel 8

### 1,7-Bis(2'-Thienyl)-4-(4'-nitrobenzyl)-2,6-diaza-hepta-1,6-dien [50]

Diese Verbindung wird entsprechend der Verbindung [4] dargestellt
Ausbeute: 91 %
¹H-NMR-Daten in CDCl₃/TMS
1,4 ppm (m,1H,CH); 3,2 ppm (d,2H,CH₂Ar); 3,5 ppm (m,4H,CH₂N=C); 6,9 ppm (m,4H,ArH); 7,2-7,4 ppm (m,4H,ArH); 8,2 ppm (d,2H,ArH); 8,3 ppm (s,2H,CH=N)

### 1,7-Bis(2'-Thienyl)-4-(4'-nitrobenzyl)-2.6-diaza-heptan [51]

Diese Verbindung wird entsprechend der Verbindung [5] dargestellt
Ausbeute: 76 %
¹H-NMR-Daten in CDCl₃/TMS
1,4 ppm (m,1H,CH); 2,5 ppm (m,4H,CH₂NH); 2,8 ppm (d,2H,CH₂Ar); 3,8 ppm (m, 4H,ArCH₂NH); 6,7 ppm (m,2H,ArH); 6,8 ppm (m,4H,ArH), 7,4 ppm (m,2H,ArH); 8,2 ppm (d,2H,ArH)

### 1,7-Bis(2'-Thienyl)-4-(4'-aminobenzyl)-2,6-diaza-heptan [52]

Diese Verbindung wird entsprechend der Verbindung [6] dargestellt
Ausbeute: 88 %
¹H-NMR-Daten in DMSO/TMS
1,2 ppm (m,1H,CH); 2,4-2,5 ppm (m,6H,CH₂NH+CH₂Ar); 3,8 ppm (m,4H, ArCH₂NH); 6,5-6,6 ppm (m,4H,ArH); 6,8-6,9 ppm (m,6H,ArH)

### Beispiel 9

### 2-Methyl-2-(2-propinyl)-malonsäuredimethylester [53]

Diese Verbindung wird entsprechend der Verbindung [1] dargestellt.
Ausbeute: 66%
¹H-NMR-Daten in CDCl₃/TMS
1,5 ppm (s,3H,Me); 2,0 ppm (t,1H,CCH); 2,8 ppm (m,2H,CH₂); 3,8 ppm (s,6H,COOMe)

### 2-Methyl-2-(2-propinyl)-malonsäurediamid [54]

Diese Verbindung wird entsprechend der Verbindung [2] dargestellt.
Ausbeute: 72%
¹H-NMR-Daten in DMSO/TMS
1,5 ppm (s,3H,Me); 1,9 ppm (m,1H,CCH); 2,6 ppm (m,2H,CH₂)

### 2-Methyl-2-(2-propinyl)-1,3-propandiamin [55]

Diese Verbindung wird entsprechend der Verbindung [36] dargestellt.
Ausbeute: 57%
¹H-NMR-Daten in CDCl₃/TMS
1,5 ppm (s,3H,Me); 1,9 ppm (m,1H,CCH); 2,5 ppm(m,6H, CH₂C+CH₂N)

### 2-Methyl-2-(propinyl)-N,N'-propylen-bis(salicylidenimin) [56]

Diese Verbindung wird entsprechend der Verbindung [4] dargestellt.
Ausbeute: 77%
¹H-NMR-Daten in CDCl₃/TMS
1,5 ppm (s,3H,Me); 2,0 ppm (t,1H,CCH); 2,6 ppm (m,2H,CH₂); 3,7 ppm (m,4H,CH₂N); 6,9-7,4 ppm (m,8H,ArH); 8,3 ppm (s,2H, CH=N)

### 2-Methyl-2-(propinyl)-N,N'-propylen-bis(salicylidenamin) [57]

Diese Verbindung wird entsprechend der Verbindung [5] dargestellt.
Ausbeute: 78%
¹H-NMR-Daten in CDCl₃/TMS
1,5 ppm (s,3H,Me); 2,0 ppm (t,1H,CCH); 2,6-2,8 ppm (m,6H,CH₂CC+ CH₂NH); 4,0 ppm (m, 4H, ArCH₂NH); 6,9-7,4 ppm (m,8H,ArH)

### Beispiel 9a

### 2-Methyl-2-(2-propinyl)-malonsäure [58]

Es werden 23.7 g [53] (127 mmol) in 200 ml Methanol gelöst und dazu eine Lösung von 28.3 g NaOH (708 mmol) in 50 ml Wasser langsam zugetropft. Anschließend wird 2 Stunden im Wasserbad auf 50°C erwärmt, das Lösungsmittel am Rotationsverdampfer abgezogen und der Rückstand in Wasser aufgenommen. Es wird mit halbkonzentrierter Salzsäure angesäuert und die freie Carbonsäure mit Chloroform extrahiert, gewaschen und getrocknet.
Ausbeute: 84%
¹H-NMR-Daten in DMSO/TMS
1,4 ppm (s,3H,Me); 2,6 ppm (d,2H, CH₂); 2,9 ppm (t,1H, CCH)

### 2-Methyl-2-(2-propinyl)-malonsäuredichlorid [59]

Die Mischung aus 3.40 g [58] und 8.9 g Thionylchlorid und einem Tropfen DMF wird unter Feuchtigkeitsausschluß unter Rühren zum Sieden erhitzt. Nach Beendigung der Gasentwicklung wird der Überschuß Thionylchlorid bei Raumtemperatur im Vakuum abgezogen und der Rückstand im Vakuum destilliert.
Ausbeute: 91%
Siedepunkt: 125 °C/₁₄ₘₘ

### N,N'-Bis(2-methoxybenzyl)-2-methyl-2-(propinyl)-malonsäurediamid [60]

Zu dem Gemisch aus 6,67 g 2-Methoxybenzylamin (48,6 mmol) und 5,06 g Triethylamin (50 mmol) in 100 ml Dichlormethan wird unter Ausschluß von Feuchtigkeit 4,69 g [59] (24,3 mmol) in 100 ml Dichlormethan vorsichtig zugetropft. Es wird noch 3 Stunden bei Raumtemperatur gerührt, dann zunächst Wasser zugegeben und mehrmals mit Chloroform extrahiert. Die organische Phase wird nacheinander mit 1N HCl, gesättigter Kaliumcarbonatlösung und Wasser gewaschen und über Natriumsulfat getrocknet.
Ausbeute: 64%
¹H-NMR-Daten in CDCl₃/TMS
1,5 ppm (s,3H,Me); 2,0 ppm (t,1H, CCH); 2,8 ppm (d,2H,CH₂CC); 3,8 ppm (s,6H, OMe); 4,4 ppm (d,4H,CH₂NH); 6,9-7,3 ppm (m,8H,ArH)

### N,N'-Bis(2-methoxybenzyl)-2-methyl-2-(propinyl)-1,3-propandiamin [61]

Diese Verbindung wird entsprechend der Verbindung [36] dargestellt.
¹H-NMR-Daten in CDCl₃/TMS
1,5 ppm (s,3H,Me); 2,0 ppm (t,1H,CCH); 2,6-2,8 ppm (m,6H,CH₂CC+ CH₂NH); 3,8 ppm (s,6H,OMe); 4,2 ppm (m, 4H, ArCH₂NH); 6,9-7,3 ppm (m,8H,ArH)

### 2-Methyl-2-(propinyl)-N,N'-propylen-bis(salicylidenamin [57]

Eine Lösung von 338 mg [61] (1,0 mmol) in 15 ml Dichlormethan wird unter Ausschluß von Feuchtigkeit unter einer Stickstoff-Atmosphäre auf 0°C abgekühlt und unter Rühren mit Hilfe einer Einwegspritze 1,75 g Bortribromid (7,0 mmol) in 20 ml Dichlormethan zugetropft und über Nacht bei Raumtemperatur gerührt. Es wird mit Wasser hydrolisiert, mit gesättigter Kaliumcarbonat-Lösung schwach alkalisiert, mit Dichlormethan extrahiert und die organischen Extrakte mit gesättigter Kochsalzlösung gewaschen. Nach Abziehen des Lösungsmittels verbleibt ein fester Rückstand.
Ausbeute: 79%

### Beispiel 10

### 2-Methyl-2-[4-(biotincarbamoyl)benzyl]-N,N'-propylen-bis(salicylidenamin) [68]

In einem 5 ml Rundkolben werden bei Raumtemperatur 100 mg Hydrochlorid des Anilins [6] (0,194 mmol) in 10 ml Wasser gelöst, und dazu wird die frisch hergestellte Lösung von 66,3 mg Biotin-NHS (0,194 mmol) in 1 ml Dimethylformamid zugegeben und anschließend unter Rühren mit 80 »l Triethylamin versetzt. Nach 15 Stunden wird das Reaktionsgemisch im Vakuum eingeengt und der Rückstand in Dimethoxyethan aufgenommen. Die Reinigung erfolgt mittels HPLC. Säule: M&W 250x4,6 mm, Nucleosil 100 C-18, 5 »m; Laufmittel ([Acetonitril]/[0,05M KH₂PO₄, pH 4,8]) 50:50 (v/v); Fluß: 1,0 ml/min UV-Detektor 280 nm.
¹H-NMR-Daten in DMSO
1,1 ppm (s,3H,Me); 1,5-1,6 ppm (m,6H,CH₂); 2,5-2,8 ppm (m,8H,CH₂S, CH₂NH,CH₂CO); 2,9 ppm (s,2H,CH₂Ar); 3,1 ppm (m,1H,CHS); 4,0-4,3 ppm (m,6H,ArCH₂NH,Biotin-H); 6,4 ppm (s,2H,NH); 6,8-7,8 ppm (m,12H, ArH)

### Tc-99m-Komplex von [68]

25 »g der Verbindung [68] in 50 »l NaCl-Lösung pH 9 werden zunächst mit 10 »l einer gesättigten Zinntartrat-Lösung versetzt und 1 mCi Tc-99m-Pertechnetat aus einem Mo99/Tc99m-Generator zugegeben. Das resultierende Reaktionsgemisch wird anschließend 5 Minuten bei Raumtemperatur stehengelassen.
Die dünnschichtchromatographische Untersuchung des Reaktionsgemisches zeigt eine vollständige Reduktion des Pertechnetats und die Komplexierung des reduzierten Technetiums durch den Liganden.

### Analyse des Tc-99m-Komplexes, HPLC

Für die Analyse werden PRP-1 HPLC-Säulen der Firma Hamilton, 150x4,1 mm, 5 um Poly(styroldivinylbenzol) Copolymer verwendet.
Laufmittel: [Acetonitril/[(KH₂PO₄ pH7 0,01M) + 1% Methanol]) 50:50 (v/v), Flußrate 1,5 ml/min. Die Detektion der Radioaktivität wird mit Hilfe eines HPLC Radioaktivitätsmonitor LB 506 C-1 der Fa. Berthold durchgeführt. Die erhaltene radiochemische Reinheit des Tc-99m-Komplexes ist größer als 92 %.

### Beispiel 11

### Tc-99m-Komplex von [7]

Eine Lösung von 5,0 mg [6] in Wasser wird mit 0,1N NaOH auf pH 11 gebracht und anschließend 1 ml EtOH dazugegeben und auf 10 ml mit Wasser aufgefüllt. 400 »l dieser Lösung werden mit 80 »l einer gesättigten Zinntartrat-Lösung und 8 mCi Tc-99m-Pertechnetat aus einem Mo99/Tc99m-Generator zugegeben und 5 Minuten stehen gelassen. Anschließend wird diese Lösung je dreimal mit 2 ml Chloroform extrahiert, die organische Phase über eine kurze Natriumsulfatsäule getrocknet, mit 50 »l Thiophosgen versetzt und 5 Minuten bei Raumtemperatur inkubiert. Anschließend wird mit 100 »l Isopropanol versetzt, mit Argon das Chloroform abgedampft und zu dem Rückstand 900 »l einer 1% igen PVP-Lösung in Wasser gegeben.

### Beispiel 12

### Kopplung und Markierung eines Isothiocyanat-haltigen Chelatbildners an Proteine

Am Beispiel von F(ab)₂-Fragmenten des monoklonalen Antikörpers 17-1A soll die Kopplung von Isothiocyanat-haltigen Tc-Chelatbildnern [Verbindung 7] an Proteine beschrieben werden. Anstelle der Antikörper-Fragmente kann jedes andere Protein bzw. eine Aminogruppen-haltige Substanz verwendet werden.

Der monoklonale Antikörper 17-1A wird entsprechend literaturbekannter Methoden nach Applikation von 10⁷ der entsprechenden Hybridomazellen in den Bauchraum einer Balb/c-Maus und Aspiration der Ascitesflüssigkeit nach 7 - 10 Tagen gewonnen. Die Reinigung erfolgt nach ebenfalls literaturbekannten Methoden durch Ammoniumsulfatfällung und Affinitätschromatographie über Protein A-Sepharose. Der gereinigte Antikörper (10 mg/ml) wird bei pH 3,5 für 2 Std. mit 25 »g/ml Pepsin behandelt und anschließend mittels FPLC isoliert. Vor der Kopplung mit dem Chelatbildner werden die Fragmente bei 4 °C für 12 - 24 Std. gegen 0,1 M KH₂PO₄ /0,1 M NaHCO₃, pH 8,5, dialysiert. Die Proteinkonzentration wird auf 10 mg/ml eingestellt. Ein 5facher molarer Überschuß des NCS-haltigen Chelatbildners [Beispiel 1] wird in möglichst wenig des gleichen Puffers gelöst und zur Proteinlösung hinzugegeben. Zur Konjugatbildung wird die Mischung für 3 Std. bei 37 °C inkubiert. Anschließend wird das Konjugat für 24 - 48 Std. mit mehrfachem Pufferwechsel gegen PBS (Phosphat-gepufferte Saline) dialysiert und die Proteinkonzentration danach nötigenfalls wieder auf 10 mg/ml eingestellt. Bis zur Markierung mit Tc-99m kann das Konjugat nach Sterilfiltration bei 4 °C in säuregereinigten Glasgefäßen aufbewahrt werden.

Die Markierung von 1 mg des mit dem Chelatbildner [7] gekoppelten Antikörper-Fragments mit Tc-99m erfolgt durch Zugabe von 10 mCi Pertechnetatlösung (= 1 - 2 ml) und 100 »g Zinn-II-chlorid in einer Argon-gespülten Na-Pyrophosphat-Lösung (1mg/ml) oder durch Ligandenaustausch z.B. durch Zugabe der Lösung eines mit Pertechnetat versetzten kommerziell erhältlichen Glucoheptonat-Kits.

### Beispiel 13

### Kopplung eines Isothiocyanat-haltigen Tc-99m-Komplexes an Proteine

Die Kopplung an die Antikörper-Fragmente kann auch nach Komplexierung der Chelatbildner mit Tc-99m durchgeführt werden. Der monoklonale Antikörper 17-1A wird wie in Beispiel 12 beschrieben gewonnen. Die Reinigung erfolgt ebenfalls wie in Beispiel 12 durch Ammoniumsulfatfällung und Affinitätschromatographie über Protein A-Sepharose. Der gereinigte Antikörper (10 mg/ml) wird bei pH 3,5 für 2 Std. mit 25 »g/ml Pepsin behandelt und anschließend mittels FPLC isoliert. Vor der Kopplung mit dem Chelatbildner werden die Fragmente bei 4 °C für 12 - 24 Std. gegen 0,1 M KH₂PO₄ / 0,1 M NaHCO₃, pH 8,5, dialysiert. Die Proteinkonzentration wird auf 10 mg/ml eingestellt. Der analog Beispiel 14 hergestellte Tc-99m-Komplex wird in einem molaren Verhältnis 1 : 10 (Komplex : Protein) zur Proteinlösuung hinzugegeben. Zur Konjugatbildung wird die Mischung für eine Stunde bei 37 °C inkubiert. Die Organverteilung in Nacktmäusen wird analog Beispiel 14 durchgeführt uns liefert ähnlich Ergebnisse.

### Beispiel 14

### Bioverteilung eines Protein-gebundenen Tc-99m-Komplexes

Am Beispiel eines mit Tc-99m markierten Konjugates aus F(ab)2-Fragmenten des monoklonälen Antikörpers 17-1A und der in Beispiel 1 hergestellten Verbindung [7] soll die Bioverteilung von Protein-gebundenen Chelaten beschrieben werden. Der Antikörper, aus dem die Fragmente gewonnen werden, erkennt ein Antigen, das von der menschlichen Carcinomzellinie "HT-29" exprimiert wird. Eine Kontrollzellinie, die ebenfalls aus einem menschlichen Carcinom (MX-1) gewonnen wird, exprimiert dieses Antigen nicht. Isolierte Zellen beider Linien werden immundefizienten Nacktmäusen subcutan appliziert. Nachdem die Tumoren zu einer Größe von 300 - 800 mg herangewachsen sind, werden den Mäusen intravenös 20 »g des mit 200 »Ci markierten Chelat-Konjugates [Beispiel 12] verabreicht. Die Konjugate werden zuvor durch eine Gelfiltration auf PD 10-Säulen der Fa. Bio-Rad von niedermolekularen Bestandteilen befreit. Die Immunreaktivität der Konjugate wird mittels der Bindung an einen Überschuß von zellständigem Antigen bestimmt und beträgt 70 - 80 %. Die Bioverteilung wird 24 Std. nach Applikation des Konjugates durch Tötung der Tiere, Entnahme der Organe und Messung der Radioaktivität in den Organen bestimmt. Die folgende Tabelle stellt die gefundenen Radioaktivitätsmengen dar und zeigt eine deutliche Anreicherung des Chelats in dem Antigen-positiven Tumor.

| **Organ** | **% der applizierten Dosis pro Gramm Gewebe** | |
|---|---|---|
| Milz | 0,3 | |
| Leber | | 1,2 |
| Nieren | | 2,8 |
| Lunge | | 0,8 |
| Muskel | 0,1 | |
| Blut | 0,6 | |
| MX-1 | 1,8 | |
| HT29 | | 12,1 |

### Beispiel 15

### Bioverteilung eines Biotin-haltigen Chelats

20 »l eines kommerziell erhältlichen Streptavidin-gekoppelten Sepharose-Gels (entsprechend 20 »g Streptavidin) werden einer 200 g schweren Ratte in den Muskel des linken Hinterbeins appliziert. Etwa 30 min danach erfolgt die intravenöse Applikation von 5 »g der in Beispiel 10 hergestellten Verbindung markiert mit 200 »Ci Tc-99m. Die Bestimmung der Radioaktivität in den einzelnen Organen der Ratte erfolgt nach 4 Stunden. Eine 14fach höhere Radioaktivität, die im linken Hinterlaufmuskel im Vergleich zum rechten Hinterlaufmuskel gefunden wird, zeigt eine deutliche spezifische Anreicherung des Tc-Komplexes durch Bindung an Streptavidin-Sepharose. In allen anderen Organen wird nach 4 Stunden keine Aktivitäten über 1% der applizierten Dosis pro Gramm Gewebe detektiert.

Die höchste Anreicherung nach dem linken Hinterlaufmuskel (1,4 % der applizierten Dosis pro Gramm Gewebe) findet sich in den Nieren mit 0,6% der appl. Dosis pro Gramm Gewebe. Etwa 93 % der applizierten Radioaktivität werden nach 4 Stunden im Harn gefunden.

### Beispiel 16

### Bindung des in Beispiel 5 hergestellten Tc-99m-Komplexes an hypoxische Zellaggregate

Die Bindung des Tc-markierten Misonidazol-Derivates wird in vitro an Zellen des Morris Hepatoms 7777 nachgewiesen. Diese Zellen besitzen die Eigenschaft in Form von Sphäroiden bzw. großen flotierenden Zellaggregaten zu wachsen und ähneln somit einem Tumor mehr als Einzelzellen. Für eine Inkubation bei verschiedenen Sauerstoffkonzentrationen werden die Zellen in 60 mm-Kulturschalen ausgesät und die Schalen in ein gasdicht verschließbares auf 37 °C erwärmtes Glasgefäß gestellt. Das Gefäß wird 30 min gut mit einer Mischung aus N₂, 5% CO₂ und 0% bzw. 10% O₂ gespült. Anschließend werden durch eine Silikonmembran mittels einer Injektionskanüle 50 »mol/l der mit 50»Ci Tc-99m markierten Verbindung [Beispiel 5] hinzugegeben und für eine gute Durchmischung gesorgt. Nach einer Stunde wird das Gefäß geöffnet, die Zellaggregate durch Zentrifugation vom Nährmedium getrennt, einmal mit Nährmedium gewaschen und eine weitere Stunde in Nährmedium ohne Zusatz der Verbindung [Beispiel 5] in normaler Atmosphäre unter Zusatz von 5% CO₂ bei 37 °C inkubiert. Anschließend werden die Zellen isoliert und die Radioaktivität bezogen auf die Proteinmenge bestimmt. Es zeigt sich, daß bezogen auf die gleiche Proteinmenge eine etwa 2,8-fach höhere Radioaktivitätsmenge in den ohne Sauerstoff inkubierten Zellen zu finden ist. Dies zeigt die Anreicherung der Verbindung [Beispiel 5] in hypoxischen Zellen.

## Patentansprüche

1. Verbindungen der allgemeinen Formel I 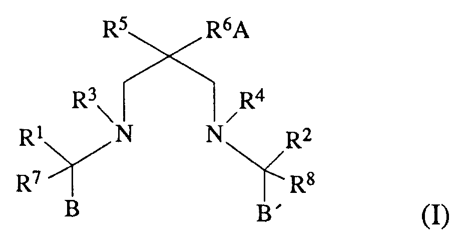 worin R¹, R² und R⁵ gleich oder unterschiedlich sind und für Wasserstoff oder einen gegebenenfalls mit einer Hydroxylgruppe substituierten C₁₋₆-Alkylrest,
R³ und R⁴ gleich oder unterschiedlich sind und für ein Wasserstoffatom, einen C₁₋₆-Alkyl- oder einen Amino(C₁₋₆)-alkylrest,
R⁶ für einen C₁₋₆-Alkylenrest,
R⁷ und R⁸ gleich oder unterschiedlich sind und für Wasserstoff oder einen C₁₋₆-Alkylrest und
B und B' gleich oder unterschiedlich sind und für einen mit 1-3 Hydroxylgruppen substituierten Phenyl- oder Naphthyl-, 2-Mercaptophenyl-, Thienyl-, Pyrrolylrest stehen,
und A eine funktionelle Gruppe C darstellt,
wobei C für einen Amino-, einen Hydrazino- oder Hydrazido-, einen Carboxy-, einen C₂₋₆-Alkinyl- oder -Alkenyl-, einen Hydroxyl-, einen Aminophenyl-, einen Oxiranyl-, einen fluorierten Phenoxycarbonyl-, einen Nitril-, einen Isothiocyanatophenyl- oder einen gegebenenfalls mit einem Natriumsulfatrest substituierten Succinimidoxycarbonylrest steht,
oder eine - mit Hilfe der funktionellen Gruppe C gebundene - sich selektiv in Läsionen oder bestimmten Geweben anreichernde Verbindung T enthält,
wobei T für monoklonale Antikörper oder deren Fragmente, Hormone, Enzyme, Wachstumsfaktoren, Liganden für Zellmembranrezeptoren, Steroide, Neurotransmitter, Lipide, Sacharide, Aminosäuren und Oligopeptide, Biotin, sowie Radiosensitizer, wie z.B. Misonidazol steht,
deren Komplexe mit - zur Diagnostik und Tumortherapie geeigneten - radioaktiven Metallionen, sowie deren Salze mit anorganischen und organischen Säuren.

2. Verbindungen nach Anspruch 1, **dadurch gekennzeichnet,** daß R¹, R², R⁷ und R⁸ für Wasserstoffatome oder Methylreste stehen.

3. Verbindungen nach Anspruch 1 und 2, **dadurch gekennzeichnet,** daß A als funktionelle Gruppe C einen Carboxy-, einen Amino-, einen C₂₋₆-Alkenyl-, einen C₂₋₆-Alkinyl-, einen Nitril-, einen Oxiranyl-, einen Aminophenyl- oder einen Isothiocyanatophenylrest darstellt oder eine mit Hilfe von C gebundene Verbindung T vorzugsweise monoklonale Antikörper, deren Fragmente, Biotin oder Misonidazol enthält.

4. Metallchelate nach Anspruch 1 mit koordinativ gebundenen, radioaktiven Ionen von Tc, Re, Cu, Co, Ga, Y und In, bevorzugt Tc und Re.

5. Pharmazeutische Mittel enthaltend mindestens ein Chelat gemäß Anspruch 1, gegebenenfalls mit den in der Galenik üblichen Zusätzen.

6. Verfahren zur Herstellung von Verbindungen der allgemeinen Formel I 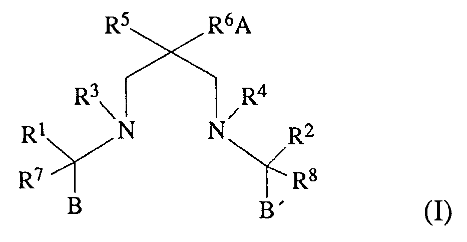 gemäß Anspruch 1
**dadurch gekennzeichnet,** daß man ein 1,3-Propandiamin der allgemeinen Formel II 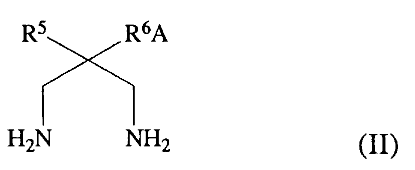 worin R⁵, R⁶ und A die oben genannten Bedeutungen haben,
mit einer Verbindung der allgemeinen Formel III bzw. IV
B - R¹C=O (III)
B'- R²C=O (IV),
in denen R¹ = R², B = B' gilt und R¹, R², B und B' die oben genannten Bedeutungen haben,
in einem polaren Lösungsmittel, vorzugsweise Ethanol, oder unter Verwendung eines Wasserabscheiders in einem unpolaren Lösungsmittel, vorzugsweise Benzol, bei Temperaturen von 25 - 180 °C innerhalb von 6 Stunden bis 3 Tagen umsetzt,
die Iminofunktion in an sich bekannter Weise, vorzugsweise mit Natriumborhydrid in einem polaren Lösungsmittel, vorzugsweise einem Methanol/Wasser-Gemisch, bei Temperaturen von 25 - 100 °C innerhalb von 0,5 bis 24 Stunden, vorzugsweise 2 Stunden, reduziert,
oder
indem man ein Propandiamin der allgemeinen Formel II 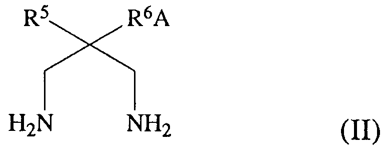 worin R⁵, R⁶ und A die oben genannten Bedeutungen haben,
mit einer Verbindung der allgemeinen Formel V bzw. VI
B - CO-X (V)
B'- CO-X (VI),
in denen B = B' gilt und B und B' die oben genannten Bedeutungen haben, in B enthaltene Hydroxyl-, Mercapto- und Aminogruppen in geschützter Form vorliegen und X für ein Halogenatom, vorzugsweise für ein Chloratom steht,
oder substituierte Malonsäurehalogenide, vorzugsweise Malonsäurechloride der allgemeinen Formel VII, 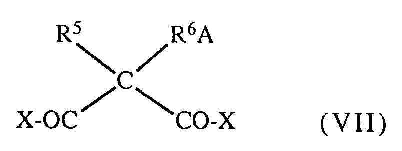 worin R⁵ und R⁶ die oben genannten Bedeutungen haben, X für ein Halogenatom steht und gegebenenfalls in B vorhandene Hydroxylgruppen in geschützter Form vorliegen,
mit einem Amin der allgemeinen Formel VIII bzw. IX 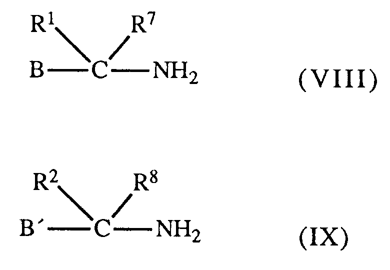 in denen R¹ = R², R⁷= R⁸, B = B' gilt und R¹, R², R⁷, R⁸,B und B' die oben genannten Bedeutungen haben,
in einem aprotischen Lösungsmittel, vorzugsweise Dichlormethan, bei Temperaturen von 0 - 180 °C, vorzugsweise bei Raumtemperatur, innerhalb von 2 bis 24 Stunden, vorzugsweise 4 Stunden, unter Zusatz von Triethylamin umsetzt,
die Amidfunktion in an sich bekannter Weise, vorzugsweise mit Boran in THF oder mit Lithiumaluminiumhydrid in einem aprotischen Lösungsmittel, vorzugsweise Diethylether, bei Temperaturen von 25 - 150 °C innerhalb von 0,5 bis 24 Stunden, vorzugsweise 8 Stunden, zur entsprechenden Aminofunktion reduziert,
die Aminogruppen gegebenenfalls in an sich bekannter Weise alkyliert und vorhandene Schutzgruppen abspaltet
und die auf diesen gleichwertigen Wegen erhaltenen Verbindungen gegebenenfalls vor Generierung der freien funktionellen Gruppe C die vorhandenen Aminogruppen mit Schutzionen, z.B. als Cu-Komplex, schützt und anschließend die so erhaltenen Verbindungen gewünschtenfalls über die in A vorhandene funktionelle Gruppe C an sich selektiv anreichernde Verbindungen T koppelt und die Aromat-Substituenten B und B' gewünschtenfalls mit dem jeweils gewünschten radioaktiven Isotop komplexiert, wobei man vorher im Produkt gegebenenfalls vorhandene Schutzionen nach an sich literaturbekannten Methoden entfernt und die Reihenfolge der Schritte Komplexierung mit Technetium- oder Rhenium-Isotopen und Kopplung an T vertauscht werden kann.

## Claims

1. Compounds of general formula (I) 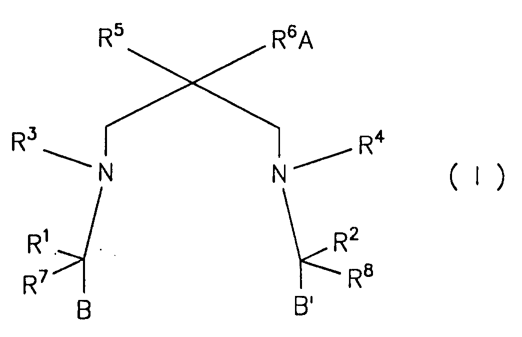 wherein
R¹, R² and R⁵ are the same or different and stand for hydrogen or a C₁₋₆-alkyl radical, optionally substituted by a hydroxyl group,
R³ and R⁴ are the same or different and stand for a hydrogen atom, a C₁₋₆-alkyl radical or an amino(C₁₋₆)-alkyl radical,
R⁶ denotes a C₁₋₆-alkylene radical,
R⁷ and R⁸ are the same or different and stand for hydrogen or a C₁₋₆-alkyl radical and
B and B' are the same or different and stand for a phenyl- or a naphtyl-, a 2-mercapto phenyl-, a thiophenyl-, or a pyrrolyl radical, substituted with 1-3 hydroxyl groups,
and A denotes a functional group C,
wherein C stands for an amino-, a hydrazine-, a hydrazide radical, a carboxyl-, a C₂₋₆-alkinyl- or a C₂₋₆-alkenyl-radical, a hydroxyl-, an aminophenyl-, an oxiranyl-, a fluorinated phenoxycarbonyl-, a nitrile-, a phenylisothiocyanate- or a succinimidoxycarbonylradical, optionally substituted by sodium sulphate,
or contains a compound T -linked through the functional group C-, which enriches selectively in lesions or defined tissues,
wherein T stands for monoclonal antibodies or fragments thereof, hormones, enzymes, growth factors, ligands for cell membrane receptors, steroids, neurotransmitters, lipids, saccharides, amino acids and oligopeptides, biotin, as well as radio sensitisers, as for example misonidazol,
complexes thereof with radioactive metal ions - suitable for diagnostic purposes and tumor therapy - and salts thereof with organic and anorganic acids.

2. Compound according to claim 1, characterized in that R¹, R², R⁷ and R⁸ denote hydrogen atoms or methyl radicals.

3. Compounds according to claims 1 and 2, characterized in that A denotes as functional group C a carboxyl radical, an amino radical, a C₂₋₆-alkenyl radical, a C₂₋₆-alkinyl radical, a nitrile radical, an oxiranyl radical, an aminophenyl radical or a phenylisothiocyanate radical or contains a compound T, which is bound through C, preferably antibodies, fragments thereof, biotin or misonidazol.

4. Metal chelates according to claim 1 with coordinatively bonded radioactive ions of Tc, Re, Cu, Co, Ga, Y and In, preferably Tc and Re.

5. Pharmaceutical means containing at least one chelate according to claim 1, optionally with additives as usual in galenic.

6. Process for the preparation of compounds of general formula (I) 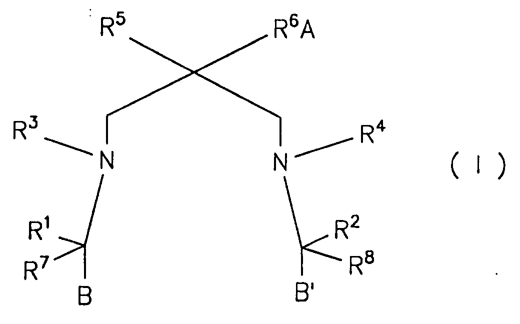 according to claim 1, characterized in that a 1,3-propane diamine of the general formula (II) 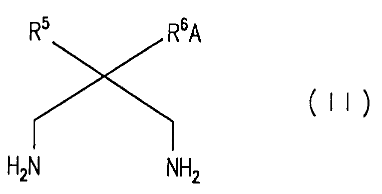 wherein R⁵, R⁶ and A have the above mentioned meanings,
is reacted with a compound of the general formula (III) or (IV)
B-R¹C=O (III)
B'-R²C=O (IV)
where R¹=R², B=B' and R¹, R², B and B' have the above mentioned meanings ,
in a polar solvent, preferably ethanol, or in a nonpolar solvent by using a water trap, preferably benzene, at temperatures of 25-180 °C within 6 hours to 3 days,
the imino function is reduced in a manner known per se, preferably by sodium boron hydride in a polar solvent, preferably in a ethanol/water-mixture, at temperatures of 25 - 100 °C within 0.5 to 24 hours, preferably 2 hours,
or a propane diamine of the general formula (II) 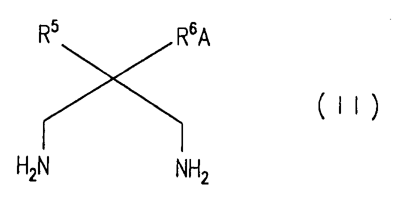 in which R⁵, R⁶ and A have the above mentioned meanings,
is reacted with a compound having the general formula (V) or (VI)
B-CO-X (V)
B'-CO-X (VI),
in which B=B', B and B' have the above mentioned meanings, hydroxyl groups, mercapto groups and amino groups being present in B are protected, and X denotes a halogen atom, preferably a chlorine atom,
or substituted malonic acid halides, preferably malonic acid chlorides, having the general formula (VII) 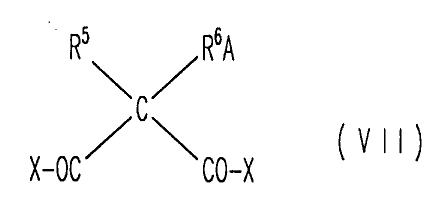 wherein R⁵ and R⁶ have the above mentioned meanings, X stands for a halogen atom and hydroxyl groups optionally present in B are protected,
are reacted with an amine of the general formula (VIII) or (IX) respectively 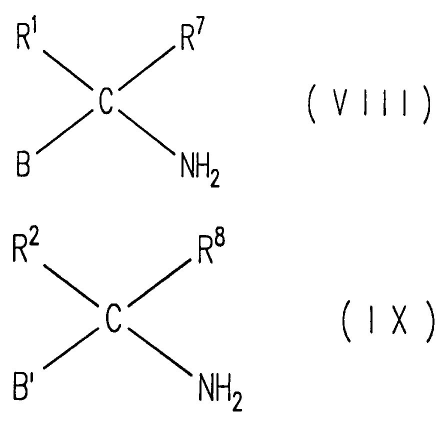 wherein R¹=R², R⁷=R⁸, B=B' and R¹, R², R⁷, R⁸, B and B' have the above mentioned meanings,
in an aprotic solvent, preferably dichlor methane, at temperatures of 0-180 °C, preferably at room temperature, within 2 to 24 hours, preferably 4 hours, under addition of triethylamine,
the amide function is reduced to the corresponding amino function in known manner, preferably with borane in THF or with lithiumaluminum hydride in an aprotic solvent, preferably diethylether, at temperatures of 25 - 150 °C within 0.5 to 24 hours, preferably 8 hours,
the amino groups are optionally alkylated in a manner known per se and existing protective groups are split off
and amino groups existing in the compounds obtained in this equivalent ways are protected if necessary before generation of the free functional group C by protective ions, for example as a copper complex, and the obtained compounds are in the following optionally coupled to the selectively enriching compound T through the functional group C present in A and the aromatic substituents B and B' are optionally complexed with a desired radioactive isotop, whereby protective ions present in the compound have to be removed first in a manner known per se and the order of the steps complexation with technetium isotopes or rhenium isotopes and the coupling to T may be inversed.

## Revendications

1. Composés de formule générale I 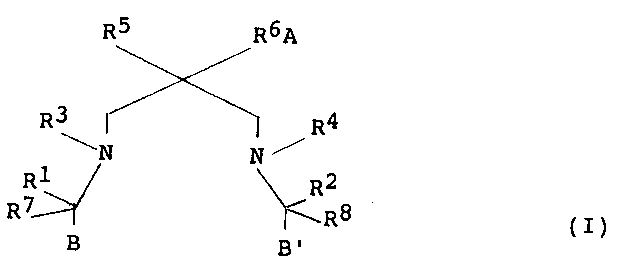 dans laquelle R¹, R² et R⁵ sont identiques ou différents et représentent un atome d'hydrogène ou un radical alkyle en C₁-C₆, éventuellement substitué par un groupe hydroxy,
R³ et R⁴ sont identiques ou différents et représentent un atome d'hydrogène, un radical alkyle en C₁-C₆ ou amino-(C₁-C₆)alkyle,
R⁶ représente un radical alkylène en C₁-C₆,
R⁷ et R⁸ sont identiques ou différents et représentent un atome d'hydrogène ou un radical alkyle en C₁-C₆, et
B et B' sont identiques ou différents et représentent un radical phényle ou naphtyle substitué par 1 à 3 groupes hydroxy, un radical 2-mercaptophényle, thiényle, pyrrolyle,
et A est un groupe foncionnel C,
C représentant un radical amino, hydrazino ou hydrazido, carboxy, alcynyle en C₂-C₆ ou alcényle en C₂-C₆, hydroxy, aminophényle, oxirannyle, phénoxycarbonyle fluoré, nitrile, isothiocyanatophényle ou un radical succinimidoxycarbonyle éventuellement substitué par un radical sulfate de sodium,
ou contient un composé T, lié à l'aide du groupe fonctionnel C, ledit composé T se concentrant de manière sélective dans des lésions ou des tissus spécifiques,
T représentant des anticorps monoclonaux ou des fragments de ceux-ci, des hormones, des enzymes, des facteurs de croissance, des ligands pour récepteurs de membrane cellulaire, des stéroïdes, des neurotransmetteurs, des lipides, des saccharides, des amino-acides et des oligopeptides, la biotine, ainsi que des agents radiosensibilisants comme, par exemple, le misonidazole,
leurs complexes avec des ions de métaux radioactifs convenant pour le diagnostic et la thérapie des tumeurs, ainsi que leurs sels formés avec des acides organiques et inorganiques.

2. Composés selon la revendication 1, caractérisés en ce que R¹, R², R⁷ et R⁸ représentent des atomes d'hydrogène ou des radicaux méthyle.

3. Composés selon les revendications 1 et 2, caractérisés en ce que A représente, en tant que groupe fonctionnel C, un radical carboxy, amino, alcényle en C₂-C₆, alcynyle en C₂-C₆, nitrile, oxirannyle, aminophényle ou isothiocyanatophényle, ou contient un composé T, lié à l'aide de C, ledit composé T représentant, de préférence, des anticorps monoclonaux, des fragments de ceux-ci, la biotine ou le misonidazole.

4. Chélates métalliques selon la revendication 1 formés avec des ions radioactifs, liés par coordination, de Tc, Re, Cu, Co, Ga, Y et In, de préférence, de Tc et Re.

5. Agent pharmaceutique contenant au moins un chélate selon la revendication 1, avec, éventuellement, les additifs courants de la galénique.

6. Procédé pour fabriquer des composés de formule générale I 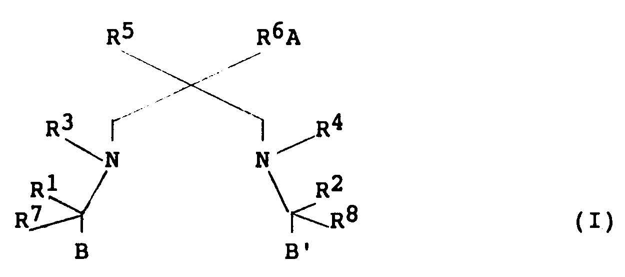 selon la revendication 1,
caractérisé en ce que l'on fait réagir une 1,3-propanediamine de formule générale II 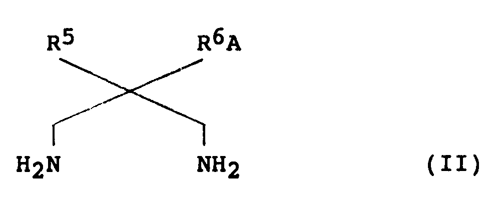 dans laquelle R⁵, R⁶ et A ont les significations précédemment citées,
avec un composé de formules générales III ou IV
B - R¹C=O (III),
B'- R²C=O (IV),
dans lesquelles R¹ = R², B = B' et R¹, R², B et B' ont les significations précédemment citées,
dans un solvant polaire, de préférence, l'éthanol, ou en utilisant un séparateur d'eau dans un solvant non polaire, de préférence, le benzène, à des températures valant de 25 à 180°C, en un laps de temps allant de 6 heures à 3 jours,
on réduit la fonction imino, de manière connue en soi, de préférence, avec du borohydrure de sodium dans un solvant polaire, de préférence, un mélange méthanol/eau, à des températures valant de 25 à 100°C, en un laps de temps allant de 0,5 à 24 heures, de préférence, en 2 heures,
ou
en ce que l'on fait réagir une propanediamine de formule générale II 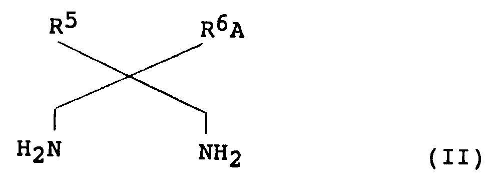 dans laquelle R⁵, R⁶ et A ont les significations précédemment citées,
avec un composé de formules générales V ou VI
B - CO-X (V),
B'- CO-X (VI),
dans lesquelles B = B', et B et B' ont les significations précédemment citées, les groupes hydroxy, mercapto et amino contenus dans B étant présents sous forme protégée et X représentant un atome d'halogène, de préférence, un atome de chlore,
ou des halogénures d'acide malonique substitués, de préférence, des chlorures d'acide malonique, de formule générale VII 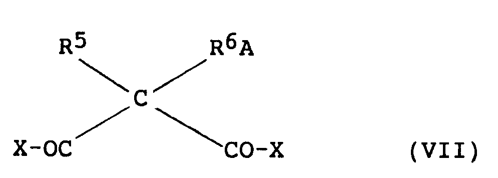 dans laquelle R⁵ et R⁶ ont les significations précédemment citées, X représente un atome d'halogène et les groupes hydroxy existant éventuellement dans B sont présents sous forme protégée,
avec une amine de formule générale VIII ou IX 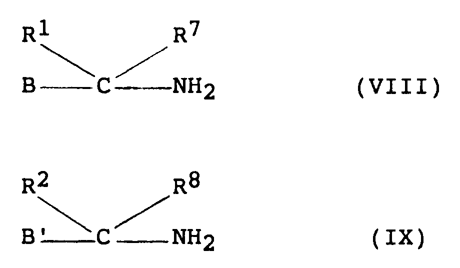 dans lesquelles R¹ = R², R⁷ = R⁸, B = B', et R¹, R², R⁷, R⁸, B et B' ont les significations précédemment citées,
dans un solvant aprotique, de préférence, le dichlorométhane, à des températures valant de 0 à 180°C, de préférence, à la température ambiante, en un laps de temps allant de 2 à 24 heures, de préférence, en 4 heures, en ajoutant de la triéthylamine,
on réduit la fonction amide en la fonction amino correspondante, de manière connue en soi, de préférence, avec du borane dans le THF ou avec de l'aluminohydrure de lithium dans un solvant aprotique, de préférence, l'éther diéthylique, à des températures valant de 25 à 150°C, en un laps de temps allant de 0,5 à 24 heures, de préférence, en 8 heures,
on effectue éventuellement l'alkylation des groupes amino, de manière connue en soi, et on élimine les groupes protecteurs,
et, avec des ions protecteurs, on protége les groupes amino présents dans les composés obtenus en suivant ces voies équivalentes, éventuellement avant la formation du groupe fonctionnel C libre, par exemple, sous forme de complexe de Cu, puis, par l'intermédiaire du groupe fonctionnel C présent dans A, on couple à volonté les composés ainsi obtenus à des composés T se concentrant de manière sélective, et on effectue à volonté la complexation des substituants aromatiques B et B' avec l'isotope radioactif voulu, les ions protecteurs éventuellement présents auparavant dans le produit étant éliminés selon des méthodes connues en soi dans la littérature, et l'ordre des étapes de complexation avec des isotopes du technétium ou du rhénium et de couplage à T pouvant être inversé.
